# EUROPEAN PATENT APPLICATION

(11) **EP 3 315 112 A1**
(43) Date of publication of application: **02.05.2018**
(21) Application number: 16883775.5
(22) Date of filing: 19.12.2016
(51) Int. Cl.: A61J 3/00

(54) **INSPECTION ASSIST SYSTEM, TABLET PACKAGING DEVICE, AND PACKAGING CONTROL PROGRAM**

(30) Priority: 06.01.2016 JP 2016001146; 23.03.2016 JP 2016059256
(71) Applicant: Yuyama Mfg. Co., Ltd., Toyonaka-shi Osaka 561-0841 (JP)
(72) Inventor: TANAKA, Toru, Toyonaka-shi Osaka 561-0841 (JP)
(74) Representative: Maslanka Kubik, Dorota Irena
(86) International application number: PCT/JP2016/087842
(87) International publication number: WO 2017/119276

(57) **Abstract**

Providing an inspection support system, a tablet dispensing device, and a dispensing control program, being capable of supporting a pharmacist in an inspection operation of inspecting a result of a dispensing process performed by a tablet dispensing device, is a problem to be solved. The inspection support system includes: a first storage processing unit that stores a first photographed image of a tablet taken in a first dispensing process in which one or more tablets are packaged by a packaging material for each timing of dosage on the basis of prescription data, and a second photographed image of the tablet taken in one or more second dispensing processes that are re-performed for one or more timings of the dosage after the first dispensing process is performed, while associating the images with the prescription data; and a display processing unit capable of displaying the first photographed image and the second photographed image stored by the first storage processing unit.

## Description

### TECHNICAL FIELD

The present invention relates to an inspection support system, a tablet dispensing device, and a dispensing control program, for supporting a pharmacist in an inspection operation, the pharmacist inspecting a result of a dispensing process by the tablet dispensing device.

### BACKGROUND ART

In general, there is known a tablet dispensing device including a plurality of tablet cassettes housing various tablets, the tablet dispensing device being capable of dispensing tablets from each of the tablet cassettes on the basis of prescription data to fold the tablets in units of dosage (refer to Patent Literature 1, for example).

### CITATIONS LIST

### PATENT LITERATURE

Patent Literature 1: JP-A-2011-104077

### SUMMARY OF INVENTION

### TECHNICAL PROBLEMS

In a medical facility such as a hospital or a pharmacy, a pharmacist conducts an inspection operation for checking whether tablets folded by a tablet dispensing device are appropriate for prescription data. The tablet dispensing device may have a function of automatically inspecting propriety of a dispensing result by using a photographed image of tablets taken in a dispensing process. When a result of dispensing by the tablet dispensing device is not appropriate, some or all dispensing processes may be re-performed by the tablet dispensing device. However, conventionally, there is not known a system for supporting a pharmacist who finally inspects results of multiple dispensing processes performed by the tablet dispensing device as described above.

It is an object of the present invention to provide an inspection support system, a tablet dispensing device, and a dispensing control program, being capable of supporting a pharmacist in an inspection operation, the pharmacist inspecting a result of a dispensing process by the tablet dispensing device.

### SOLUTIONS TO PROBLEMS

An inspection support system according to the present invention includes: a first storage processing unit that stores a first photographed image of a tablet photographed in a first dispensing process in which one or more tablets are wrapped by a packaging material for each timing of dosage on the basis of prescription data, and a second photographed image of the tablet photographed in one or more second dispensing processes that are re-performed for one or more timings of the dosage after the first dispensing process is performed, while associating the images with the prescription data; and a display processing unit capable of displaying the first photographed image and the second photographed image stored by the first storage processing unit.

According to the inspection support system, the first photographed image and the second photographed image can be displayed, so that it is possible to support a pharmacist who conducts a final inspection in consideration of the first dispensing process and the second dispensing process.

A tablet dispensing device according to the present invention includes: a dispensing unit that folds one or more tablets with a packaging material on the basis of prescription data; a photographing unit that is used for photographing the tablets dispensed by the dispensing unit; and a first storage processing unit that stores a first photographed image of the tablets, photographed by the photographing unit in a first dispensing process in which one or more tablets are packaged with the packaging material for each timing of dosage by the dispensing unit on the basis of the prescription data, and a second photographed image of the tablets, photographed by the photographing unit in one or more second dispensing processes that are re-performed for the one or more timings of dosage after the first dispensing process is performed, while associating the images with the prescription data.

According to the tablet dispensing device, the first photographed image and the second photographed image are stored while being associated with the prescription data, and then the first photographed image and the second photographed image become available. As a result, it is possible to support a pharmacist who conducts a final inspection in consideration of the first dispensing process and the second dispensing process, for example.

A dispensing control program according to the present invention causes a processor mounted in a tablet dispensing device including a dispensing unit that folds one or more tablets with a packaging material on the basis of prescription data, and a photographing unit that is used to photograph the tablets folded by the dispensing unit, to perform: a first photographing step in which, in a first dispensing process in which one or more tablets are packaged with a packaging material for each timing of dosage, the tablets are photographed using the photographing unit; a first storage step in which a first photographed image photographed in the first photographing step is stored while being associated with the prescription data; a second photographing step in which the tablets are photographed using the photographing unit in one or more second dispensing processes that are re-performed for the one or more timings of dosage after the first dispensing process is performed; and a second storage step in which a second photographed image photographed in the second photographing step is stored while being associated with the prescription data.

According to the dispensing control program, the first photographed image and the second photographed image are stored while being associated with the prescription data, and then the first photographed image and the second photographed image become available. As a result, it is possible to support a pharmacist who conducts a final inspection in consideration of the first dispensing process and the second dispensing process, for example.

It is conceivable that an inspection support system according to the present invention includes: a display processing unit that causes a display to display prescription-related information including information on at least a part of the prescription data, and dispensing result information obtained before a dispensing process or after the dispensing process to identify a kind of each of medicines, after the dispensing process in which one or more medicines are packaged with a packaging material for each timing of dosage on the basis of prescription data is performed; and a re-performing processing unit that causes the dispensing process to be re-performed for the one or more timings of dosage in accordance with a predetermined re-dispensing operation after the prescription-related information and the dispensing result information are displayed by the display processing unit.

It is conceivable that the display processing unit causes at least a dose and usage in the prescription data to be displayed. It is also conceivable that the display processing unit causes at least a medicine name and a patient name in the prescription data to be displayed.

In addition, it is conceivable that an inspection support system according to the present invention includes: a display processing unit that causes a display to display at least dispensing result information obtained before a dispensing process or after the dispensing process to identify a kind of each of medicines after the dispensing process in which one or more medicines are packaged with a packaging material for each timing of dosage on the basis of prescription data are performed; and a re-performing processing unit that causes the dispensing process to be re-performed for the one or more timings of dosage in accordance with a predetermined re-dispensing operation after the prescription-related information and the dispensing result information are displayed by the display processing unit.

Further, it is conceivable that the above inspection support system further includes: any one of or both of: a photographing unit that photographs the medicine before the dispensing process or after the dispensing process, as a result of the dispensing process; and a component detector that detects components of the medicine before the dispensing process or after the dispensing process, as a result of the dispensing process, wherein the dispensing result information includes a photographed image of the medicine photographed by the photographing unit or a component detection result detected by the component detector.

It is also conceivable that an inspection support system according to the present invention includes: a re-performing processing unit that is capable of re-performing a dispensing process for one or more timings of dosage after the dispensing process in which one or more tablets are packaged with a packaging material for each timing of dosage on the basis of prescription data is performed; a verification processing unit that verifies the prescription data and a result of the dispensing process; and a storage processing unit that stores information indicating how many times the dispensing process for each prescription data or for each timing of dosage is performed, for each time of the dispensing process, and a verified result of the dispensing process, while associating the information and the result with each other.

In addition, it is conceivable that the above inspection support system further includes: a display processing unit capable of displaying a verified result of the dispensing process for each timing of dosage in each of the dispensing process, stored in the storage processing unit.

### ADVANTAGEOUS EFFECTS OF INVENTION

According to the present invention, it is possible to provide an inspection support system, a tablet dispensing device, and a dispensing control program, being capable of supporting a pharmacist in an inspection operation, the pharmacist inspecting a result of a dispensing process by the tablet dispensing device.

### BRIEF DESCRIPTION OF DRAWINGS

Fig. 1 is a diagram illustrating a configuration of an inspection support system according to an embodiment of the present invention.
Fig. 2 is an external view of a tablet dispensing device according to an embodiment of the present invention.
Fig. 3 is a schematic diagram illustrating a configuration of a tablet dispensing device according to an embodiment of the present invention.
Fig. 4 is a schematic diagram illustrating a configuration of a tablet dispensing device according to an embodiment of the present invention.
Fig. 5A illustrates an example of a tablet rotating unit of a tablet dispensing device according to an embodiment of the present invention.
Fig. 5B illustrates an example of a tablet rotating unit of a tablet dispensing device according to an embodiment of the present invention.
Fig. 6 illustrates an example of a result of dispensing by a tablet dispensing device according to an embodiment of the present invention.
Fig. 7 is a flowchart illustrating an example of a procedure of an inspection support process performed in an inspection support system according to an embodiment of the present invention.
Fig. 8 illustrates an example of a display screen displayed in an inspection support system according to an embodiment of the present invention.
Fig. 9 illustrates an example of a display screen displayed in an inspection support system according to an embodiment of the present invention.
Fig. 10 illustrates an example of a display screen displayed in an inspection support system according to an embodiment of the present invention.
Fig. 11 is a flowchart illustrating an example of a procedure of an inspection display control process performed by an inspection support system according to an embodiment of the present invention.
Fig. 12A illustrates an example of a display screen displayed in an inspection support system according to an embodiment of the present invention.
Fig. 12B illustrates an example of a display screen displayed in an inspection support system according to an embodiment of the present invention.
Fig. 13 illustrates an example of a display screen displayed in an inspection support system according to an embodiment of the present invention.
Fig. 14A illustrates an example of a display screen displayed in an inspection support system according to an embodiment of the present invention.
Fig. 14B illustrates an example of a display screen displayed in an inspection support system according to an embodiment of the present invention.
Fig. 16 illustrates an example of a display screen displayed in an inspection support system according to an embodiment of the present invention.

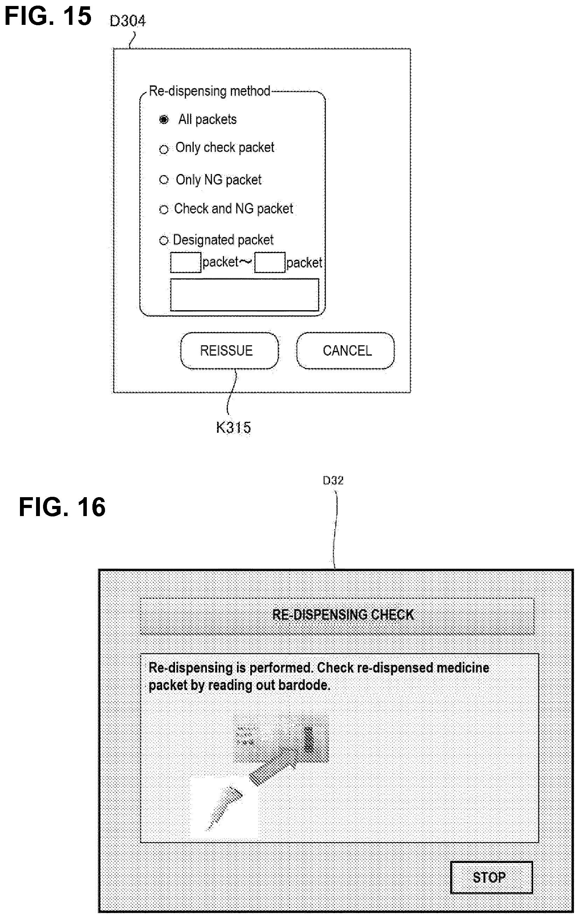

Fig. 16 illustrates an example of a display screen displayed in an inspection support system according to an embodiment of the present invention.

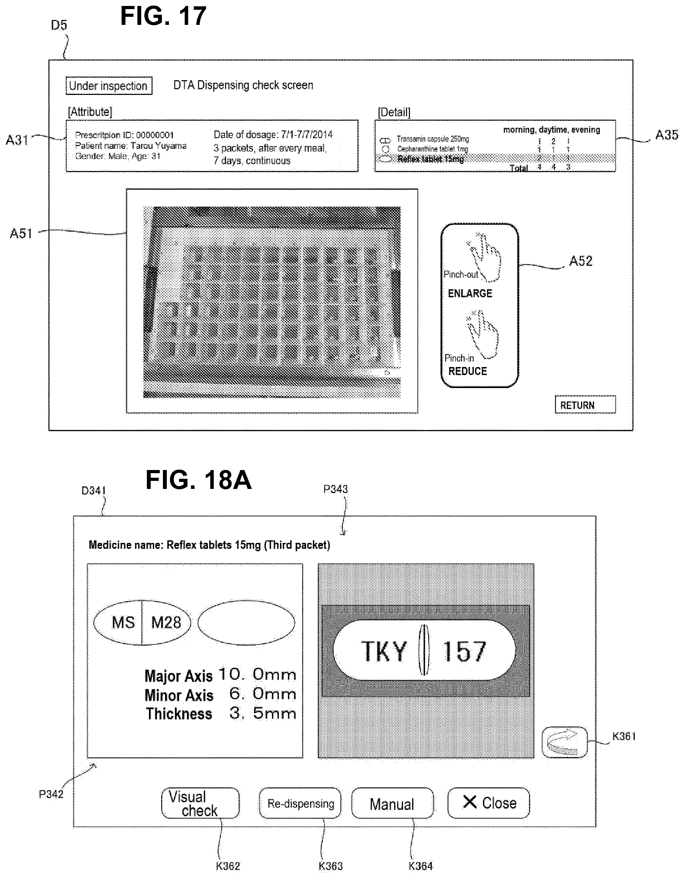

Fig. 17 illustrates an example of a display screen displayed in an inspection support system according to an embodiment of the present invention.
Fig. 18A illustrates an example of a display screen displayed in an inspection support system according to an embodiment of the present invention.

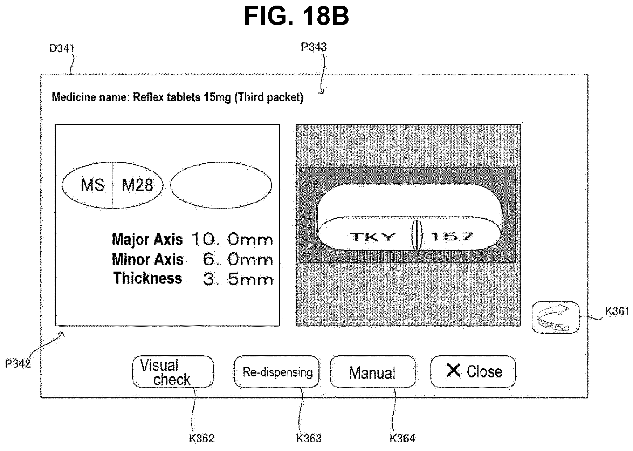

Fig. 18B illustrates an example of a display screen displayed in an inspection support system according to an embodiment of the present invention.

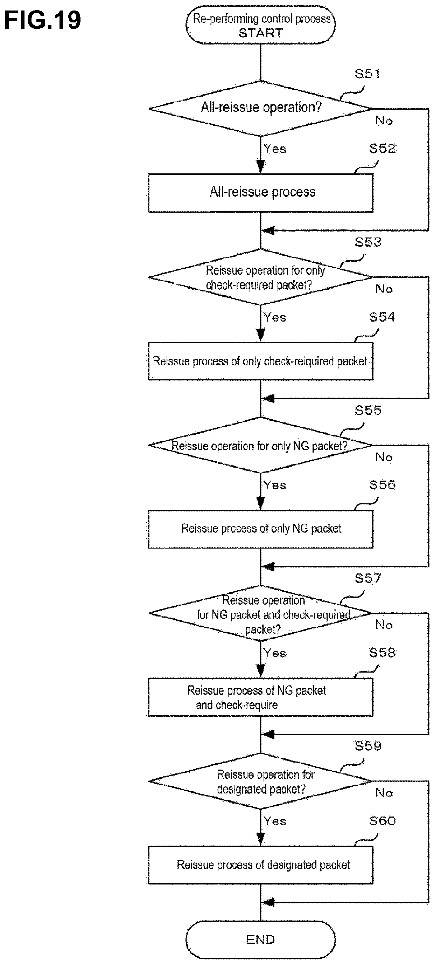

Fig. 19 is a flowchart illustrating an example of a procedure of a re-performing control process performed by an inspection support system according to an embodiment of the present invention.

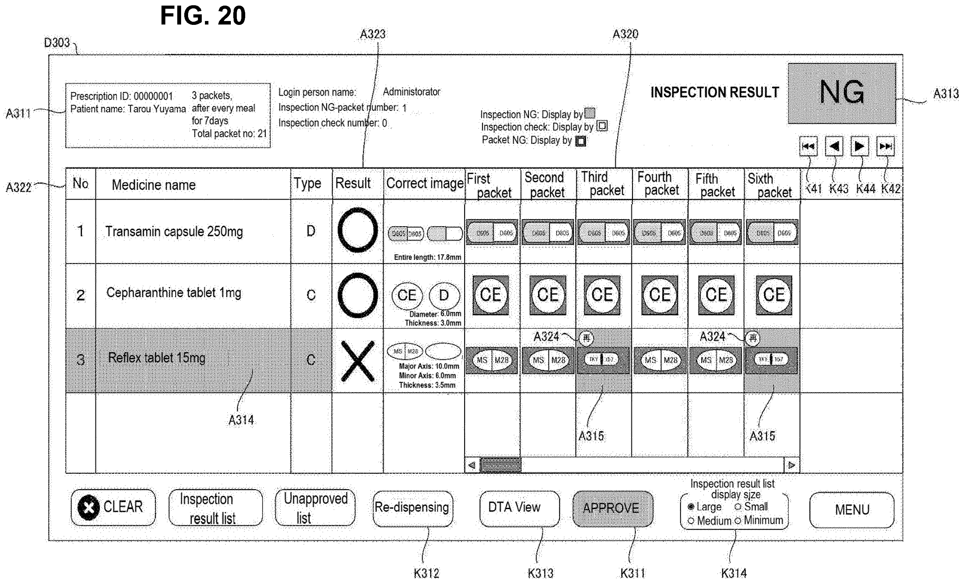

Fig. 20 illustrates an example of a display screen displayed in an inspection support system according to an embodiment of the present invention.

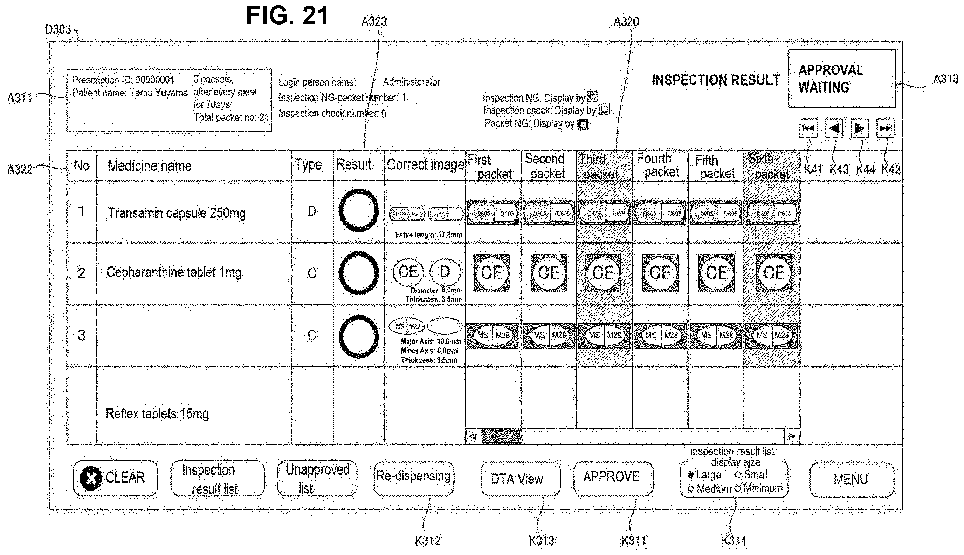

Fig. 21 illustrates an example of a display screen displayed in an inspection support system according to an embodiment of the present invention.

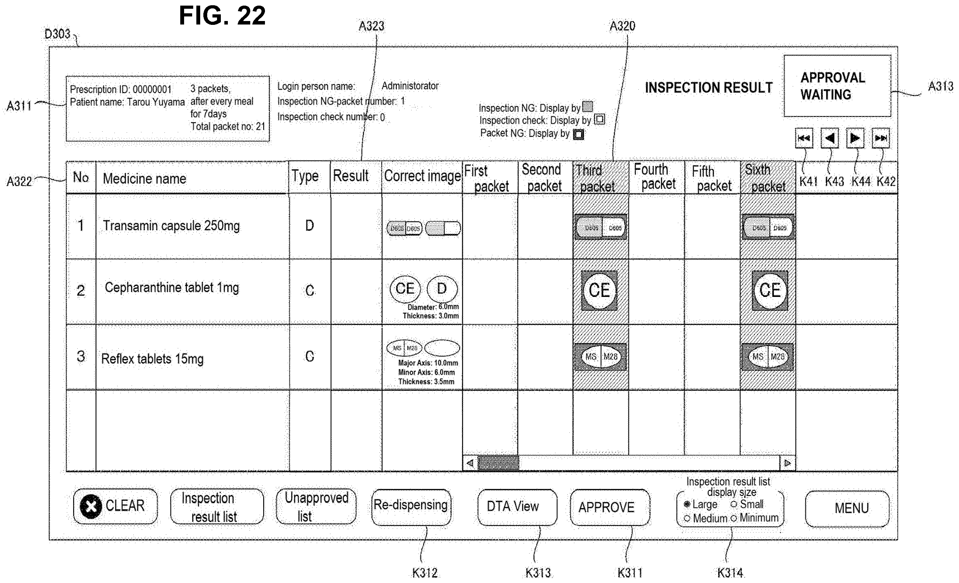

Fig. 22 illustrates an example of a display screen displayed in an inspection support system according to an embodiment of the present invention.

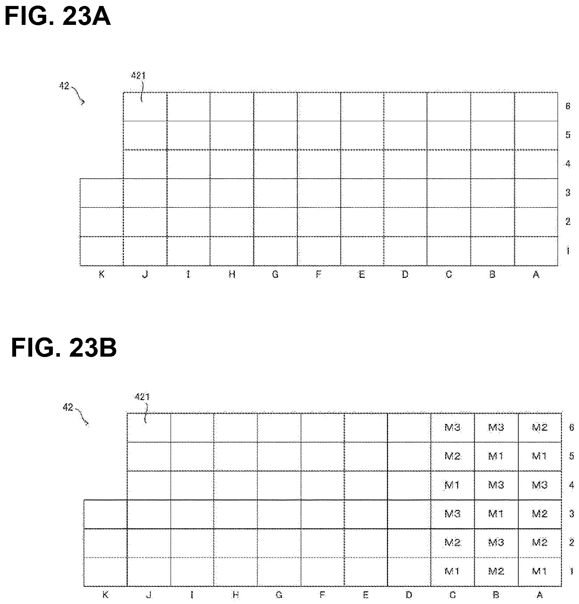

Fig. 23A illustrates an example of a display screen displayed in an inspection support system according to an embodiment of the present invention.
Fig. 23B illustrates an example of a lighting pattern in a manual dispensing unit of an inspection support system according to an embodiment of the present invention.

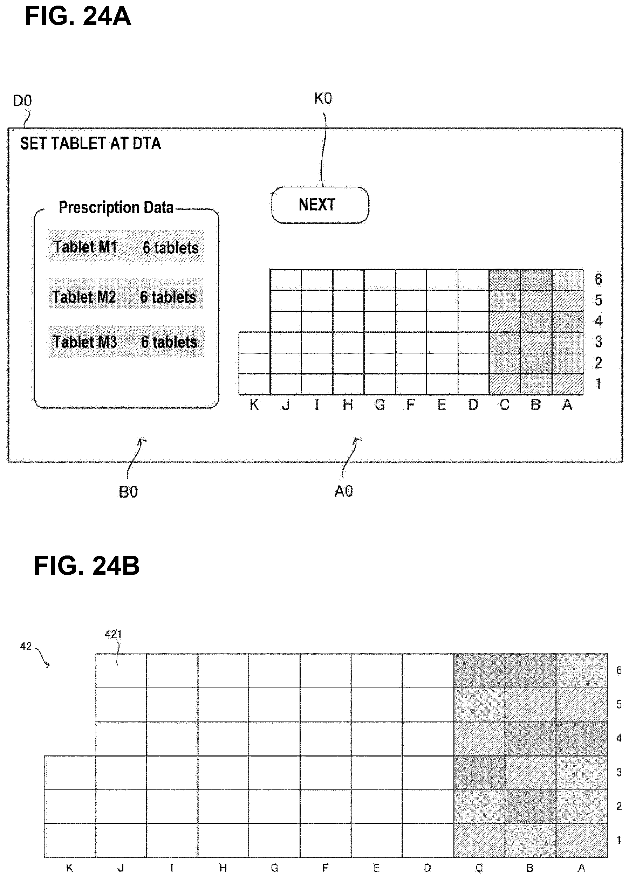

Fig. 24A illustrates an example of a display screen displayed in an inspection support system according to an embodiment of the present invention.
Fig. 24B illustrates an example of a lighting pattern in a manual dispensing unit of an inspection support system according to an embodiment of the present invention.

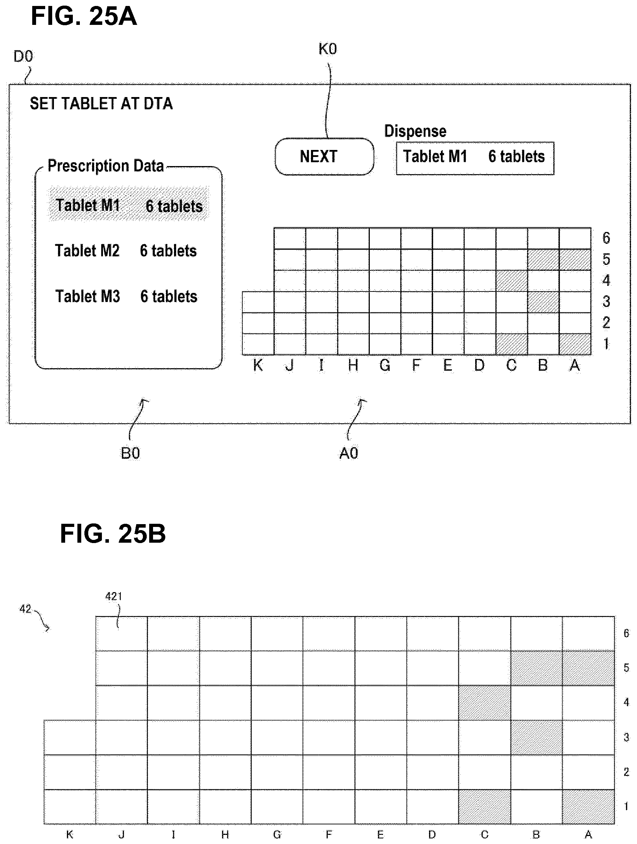

Fig. 25A illustrates an example of a display screen displayed in an inspection support system according to an embodiment of the present invention.
Fig. 25B illustrates an example of a lighting pattern in a manual dispensing unit of an inspection support system according to an embodiment of the present invention.

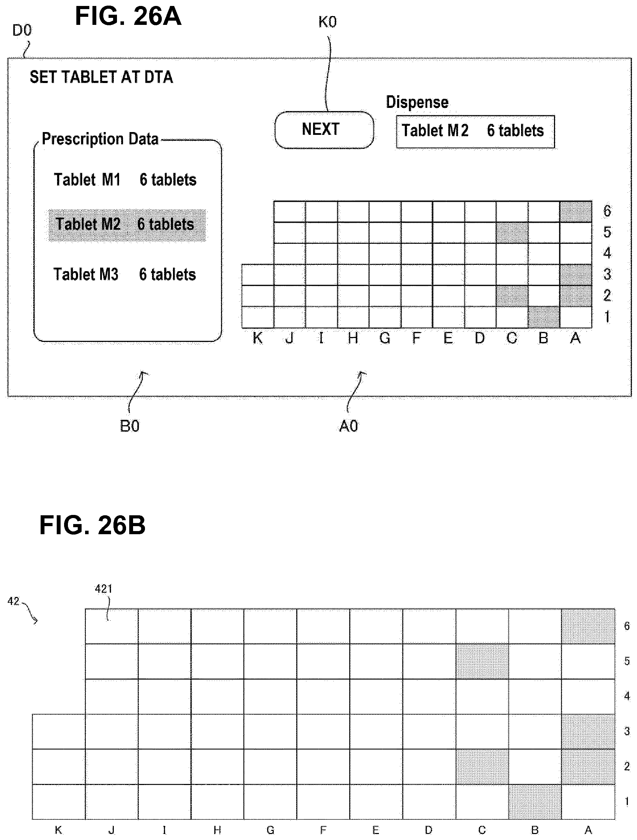

Fig. 26A illustrates an example of a display screen displayed in an inspection support system according to an embodiment of the present invention.
Fig. 26B illustrates an example of a lighting pattern in a manual dispensing unit of an inspection support system according to an embodiment of the present invention.

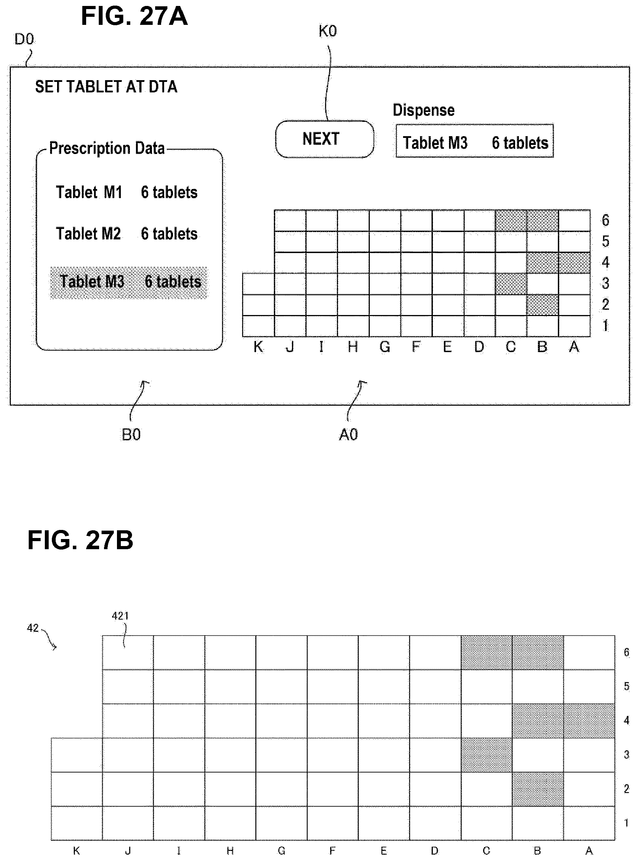

Fig. 27A illustrates an example of a display screen displayed in an inspection support system according to an embodiment of the present invention.
Fig. 27B illustrates an example of a lighting pattern in a manual dispensing unit of an inspection support system according to an embodiment of the present invention.

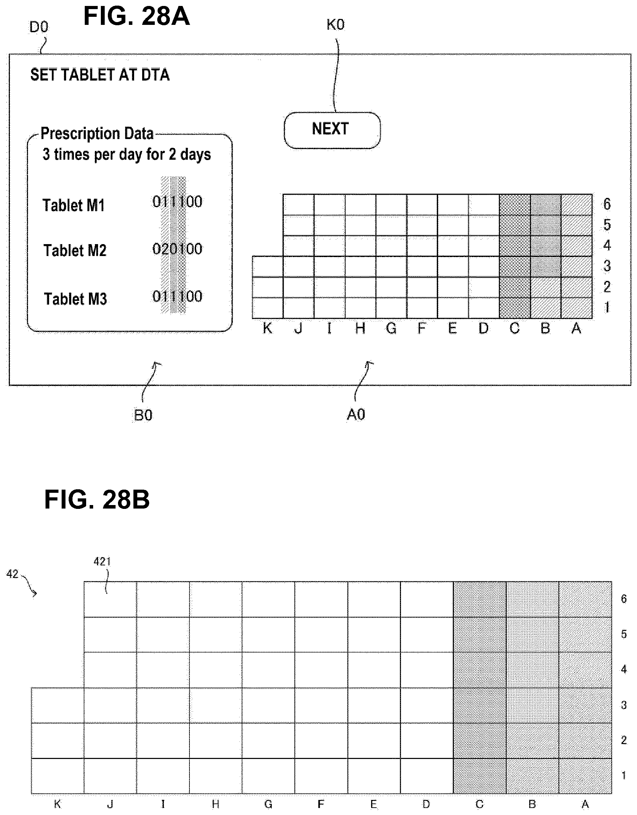

Fig. 28A illustrates an example of a display screen displayed in an inspection support system according to an embodiment of the present invention.
Fig. 28B illustrates an example of a lighting pattern in a manual dispensing unit of an inspection support system according to an embodiment of the present invention.

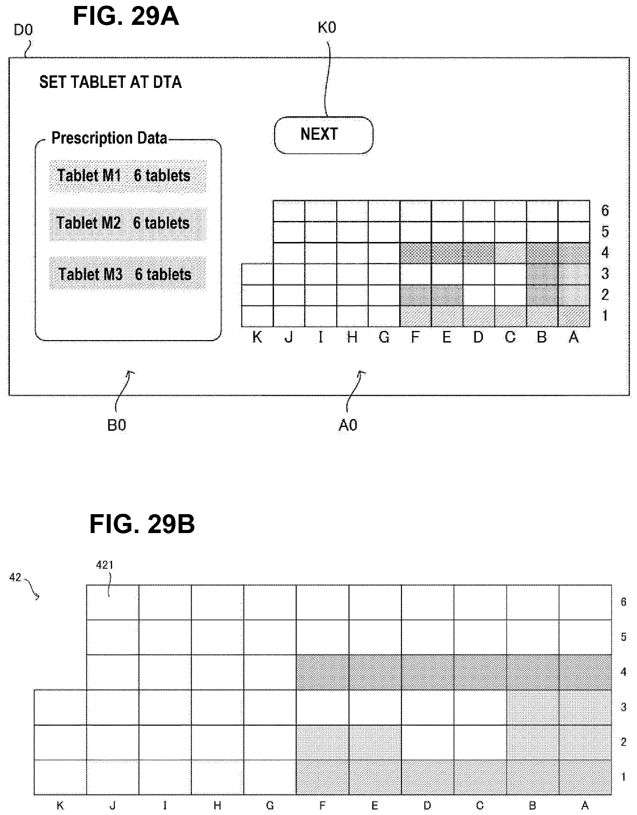

Fig. 29A illustrates an example of a display screen displayed in an inspection support system according to an embodiment of the present invention.
Fig. 29B illustrates an example of a lighting pattern in a manual dispensing unit of an inspection support system according to an embodiment of the present invention.

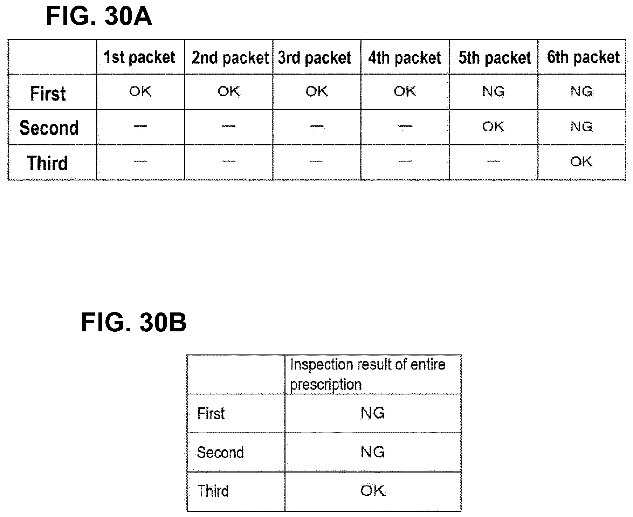

Fig. 30A illustrates an example of history information in an inspection support system according to an embodiment of the present invention.
Fig. 30B illustrates an example of history information in an inspection support system according to an embodiment of the present invention.

### DESCRIPTION OF EMBODIMENTS

Hereinafter, embodiments of the present invention will be described with reference to the accompanying drawings so that the present invention will be understood. The following embodiments are examples of embodying the present invention, and thus are not intended to limit the technical scope of the present invention. It is also possible to select some configurations and processing functions of the following embodiments to appropriately combine them.

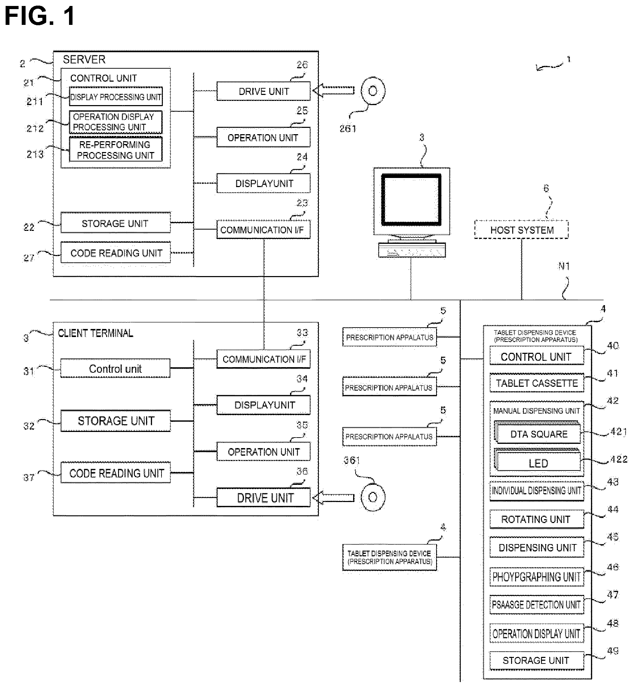

As illustrated in Fig. 1, an inspection support system 1 according to an embodiment of the present invention includes a server 2, one or more client terminals 3, one or more tablet dispensing devices 4, and one or more prescription apparatuses 5. The server 2 alone or the tablet dispensing device 4 alone may be regarded as an inspection support system according to the present invention.

The server 2, the client terminal 3, the tablet dispensing device 4, and the prescription apparatus 5, are connected to each other via a communication network N1 such as a LAN and the Internet so as to communicate with each other wirelessly or by wire. To the server 2, a host system 6, such as an electronic medical chart system or a prescription input terminal, for inputting prescription data to the server 2, is connected via the communication network N1. It is also conceivable that the server 2 can read prescription data from a prescription, or that prescription data can be input into the server 2 by a user's operation.

### [Server 2]

The server 2 is a personal computer including a control unit 21, a storage unit 22, a communication I/F 23, a display unit 24, an operation unit 25, a drive unit 26, a code reading unit 27, and the like. The server 2 is disposed inside or outside a medical facility where the inspection support system 1 is used.

The control unit 21 has control devices such as a CPU, a ROM, a RAM, and an EEPROM (registered trademark). The CPU is a processor that executes various kinds of arithmetic processing. The ROM is a nonvolatile storage unit in which information such as control programs for causing the CPU to execute various processes is stored in advance. The RAM is a volatile storage unit, and the EEPROM is a nonvolatile storage unit. The RAM and the EEPROM are each used as a temporary storage memory (a work area) of various processes executed by the CPU. The control unit 21 executes various processes by using the CPU, according to the various corresponding control programs stored in advance in the ROM, the EEPROM, or the storage unit 22.

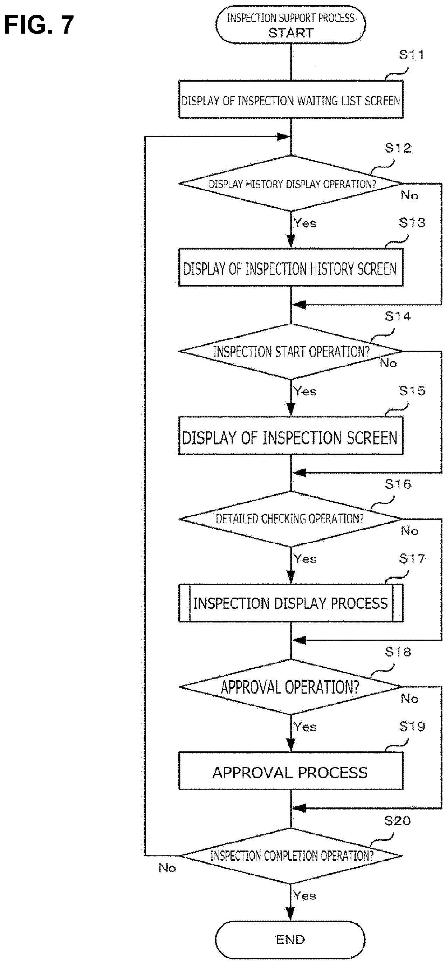

The storage unit 22 is a storage unit such as a hard disk drive or a solid state drive (SSD) that stores various data. Specifically, the storage unit 22 stores in advance an inspection support program for causing a computer such as the control unit 21 or the like to execute an inspection support process described below (refer to Fig. 7).

The storage unit 22 also stores various databases such as a medicine master, a patient master, a user master, and a cassette master, for example. The medicine master includes information about each medicine, such as a medicine ID, a medicine code, a medicine name, a YJ code, a JAN code (or an RSS code), a medicine bottle code, a classification (a dosage form, such as powder, a tablet, a liquid medicine, and an external medicine), tablet shape (e.g., a capsule tablet, a spherical tablet, and a flat tablet), tablet size, specific gravity, a type of medicine (e.g., an ordinary medicine, a poison, a narcotic, a powerful medicine, an antipsychotic, and a remedy), a blending variation, a pharmaceutical excipient, a precaution, and a correct image of a tablet (an appearance image of front and back surfaces of a tablet). The correct image is registered in advance on the basis of a photographed image of the tablet photographed by the tablet dispensing device 4, or is registered by capturing in advance the correct image registered in a medicine database described below. The user master includes information on a user, such as a pharmacy name, a pharmacist's name, a pharmacist's ID, a password, a user group, and processing authority. The patient master includes information on a patient, such as a patient ID, a name, a sex, an age, a medical history, a prescription medicine history, family information, a clinical department, a ward, and a hospital room. The cassette master is information indicating a correspondence between cassette identification information of each of medicine cassettes 41 described below of the tablet dispensing device 4, and a tablet allocated to each of the medicine cassettes 41.

In addition, the storage unit 22 stores a medicine database in which information such as a medicine code, a medicine name, a JAN code, an RSS code, a medicine bottle code, a dosage form, a unit, specific gravity, a medicine type, a blending variation, a pharmaceutical excipient, precautions, allergy information, information in attached documents, and the like, are associated with each other for each medicine, separately from the medicine master. Particularly, the medicine database stores information such as identification information on a tablet, formed on the tablet, a shape of the tablet, and the like, for each tablet. The medicine database is read out from a recording medium such as a CD or a DVD by the drive unit 26, or is received from an external device via the communication network N1, to be stored in the data storage unit 22. In the inspection support system 1, the medicine database is used when information is retrieved into various masters such as the medicine master, or when information in attached documents of each medicine is referred, for example. For example, in the inspection support system 1, the medicine database can be displayed in response to a user's operation at the server 2, the client terminal 3, or the like. In addition, the control unit 21 may be configured to read out the medicine database from an external device or from a website via the communication network N1 as required. The medicine database is used for updating the medicine master, for example.

The communication I/F 23 is a communication interface including a network card or the like for performing data communication with external devices such as the client terminal 2, the tablet dispensing device 4, and the prescription apparatus 5 via the communication network N1 wirelessly or by wire, in accordance with a predetermined communication protocol.

The display unit 24 is a display unit, such as a liquid crystal monitor, for displaying various kinds of information and operation screens in response to a control command from the control unit 21. The operation unit 25 is an operation unit such as a keyboard, a mouse, and a touch panel, for receiving a user's operation, and inputs an operation signal corresponding to a user's operation to the control unit 21. The operation unit 25 receives various operation inputs such as a selection operation of prescription data on a display screen displayed in the display unit 24, and an operation of issuing prescription data to request a start of prescribing for the prescription data.

The drive unit 26 is capable of reading the inspection support program from a computer readable recording medium 261 in which the inspection support program is recorded. The recording medium 261 is a CD, a DVD, a BD, a USB memory, or the like, and the drive unit 26 is a CD drive, a DVD drive, a BD drive, a USB port, or the like. In the server 2, the control unit 21 causes the storage unit 22 to store the inspection support program read from the recording medium 261 using the drive unit 26.

The code reading unit 27 is a bar code reader capable of reading code information (e.g., a bar code or a two-dimensional code). For example, the code reading unit 27 is used to read prescription data from code information described in a prescription. The control unit 21 causes the storage unit 22 to store the prescription data read from the prescription.

In the server 2 configured as described above, the control unit 21 includes a display processing unit 211, an operation display processing unit 212, and a re-performing processing unit 213. Specifically, the control unit 21 functions as the display processing unit 211, the operation display processing unit 212, and the re-performing processing unit 213 by performing various processes according to the inspection support program. The control unit 21 also has a function of generating prescription data for prescription (dispensing data) to cause the tablet dispensing device 4 and the prescription apparatus 5 to perform a prescription process such as a dispensing process on the basis of the prescription data, thereby inputting the prescription data to the tablet dispensing device 4 and the prescription apparatus 5. This causes the tablet dispensing device 4 and the prescription apparatus 5 to perform a prescription process such as a dispensing process on the basis of the prescription data.

The display processing unit 211 causes the client terminal 3 or the like to display a photographed image of each of tablets taken in a dispensing process in units of packet (in units of one packet), in which dispensing process one or more tablets dispensed from one or both of the tablet cassette 41 and the manual dispensing unit 42, which will be described below, are packed with a packaging material such as a medicine packet 451 for each timing of dosage in the tablet dispensing device 4 on the basis of the prescription data. While the timing of dosage is used as a term including a dosage date and a dosage time (e.g., after breakfast, after lunch, and after dinner) in the present embodiment, the timing of dosage in the present invention may merely indicate a dosage time. In particular, when the dispensing process is performed multiple times for the same prescription data, the display processing unit 211 enables photographed images taken in the dispensing process performed multiple times to be combined and displayed. The photographed images are photographed before or after each of the tablets is packed with the dispensing paper in each dispensing process. In addition, the display processing unit 211 causes the client terminal 3 or the like to display an inspection result of an automatic inspection process performed on the basis of identification information (e.g., a character or a symbol) of the tablet included in the photographed image of the tablet and the prescription data. More specifically, the display processing unit 211 enables an inspection result by the automatic inspection process when the photographed image is taken to be displayed together with the photographed image.

The operation display processing unit 212 causes an operation unit for individually re-performing some of or all of the prescription process based on the prescription data, performed by the tablet dispensing device 4 and the prescription apparatus 5, to be displayed in a screen that displays the inspection result of the medicine prescribed by the tablet dispensing device 4 and the prescription apparatus 5.

The re-performing processing unit 213 causes the tablet dispensing device 4 or the prescription apparatus 5 to perform again some of or all of the prescription process performed by the tablet dispensing device 4 or the prescription apparatus 5, in response to a user's operation on the operation unit displayed by the operation display processing unit 212. For example, the re-performing processing unit 213 creates re-performing data for performing some of or all of the prescription process on the basis of the prescription data, and transmits the re-performing data together with a re-performing command to the tablet dispensing device 4 or the prescription apparatus 5. The re-performing data is used to perform again a prescription process corresponding to one or more timings of dosage of the prescription processes based on the prescription data, and some of or all of the prescription process is performed for one or more timings of dosage.

Specifically, in the present embodiment, the operation display processing unit 212 enables a reissue operation screen D304 (refer to Fig. 15) for performing again some of or all of the dispensing process performed by the tablet dispensing device 4 to be displayed. Some of or all of the dispensing process is performed for one or more timings of dosage, and the re-performing data is used to perform again the dispensing process for one or more timings of dosage. Then, the re-performing processing unit 213 enables some of or all of the dispensing process performed by the tablet dispensing device 4 to be performed by an identical or a different tablet dispensing device 4 in response to the user's operation on the reissue operation screen D304.

### [Client Terminal 3]

The client terminal 3 is a personal computer including a control unit 31, a storage unit 32, a communication I/F 33, a display unit 34, an operation unit 35, a drive unit 36, a code reading unit 37, and the like. Each client terminal 3 is an operation terminal disposed in a medical facility where the inspection support system 1 is used, and is operated by a user such as a pharmacist.

The control unit 31 includes control devices such as a CPU, a ROM, a RAM, and an EEPROM. The CPU is a processor that executes various kinds of arithmetic processing. The ROM is a nonvolatile storage unit in which information such as control programs for causing the CPU to execute various processes is stored in advance. The RAM is a volatile storage unit, and the EEPROM is a nonvolatile storage unit. The RAM and the EEPROM are each used as a temporary storage memory (a work area) of various processes executed by the CPU. The control unit 31 executes various processes by using the CPU, according to the various corresponding control programs stored in advance in the ROM, the EEPROM, or the storage unit 32.

The storage unit 32 is a nonvolatile storage unit such as a hard disk and an SSD, storing various application programs to be executed by the control unit 31 and various data. Specifically, the storage unit 32 stores an application program such as an operating system (OS) and browser software. The browser software is application software for causing the display unit 34 to display various operation screens and the like by accessing the server 2 via the communication network N1, and transmitting an input operation on the operation screen using the operation unit 35 to the server 2. Specifically, when address information such as a universal resource locator (URL) corresponding to the server 2 is input at a predetermined position on the operation screen displayed by the browser software, the control unit 31 accesses the server 2 on the basis of the address information.

The communication I/F 33 is a communication interface including a network card or the like for performing data communication with external devices such as the server 2 via the communication network N1 wirelessly or by wire, in accordance with a predetermined communication protocol.

The display unit 34 is a display unit, such as a liquid crystal display and an organic EL display, for displaying various kinds of information in response to a control command from the control unit 31. The operation unit 35 is operated by a user to input various kinds of information to the client terminal 3. Specifically, the operation unit 35 includes a keyboard, a mouse (pointing device), a touch panel, and the like, receiving input operations on various operation screens displayed in the display unit 34. The operation unit 35 also may include a voice input device that receives input of various kinds of information by voice recognition.

The drive unit 36 is capable of reading the OS, the browser software, or the like from a computer readable recording medium 361 in which the OS, the browser software, or the like is recorded. The recording medium 361 is a CD, a DVD, a BD, a USB memory, or the like, and the drive unit 36 is a CD drive, a DVD drive, a BD drive, a USB port, or the like. In the client terminal 3, the control unit 31 causes the storage unit 32 to store the OS, the browser software, or the like, read from the recording medium 361 using the drive unit 36.

The code reading unit 37 is a bar code reader capable of reading code information (e.g., a bar code or a two-dimensional code). For example, the code reading unit 37 is used to read the code information described in the medicine packet 451.

In the inspection support system 1, the server 2 and the client terminal 3 constitute a server client system, and there will be described a case where the server 2 performs various processes in response to a user's operation of the client terminal 3. Thus, "display", "operation", "selection", "input" and the like described below are performed using the display unit 34 and the operation unit 35 of the client terminal 3. For example, the control unit 21 of the server 2 causes the client terminal 3 to display various screens by transmitting data described in a page description language such as HTML to the client terminal 3.

It is also conceivable that some or all of the inspection support program is installed in any one or more of the server 2, the client terminal 3, and the tablet dispensing device 4, and an inspection support process described below is performed on the server 2, the client terminal 3, the tablet dispensing device 4, and the like in cooperation with each other.

### [Tablet Dispensing Device 4]

The tablet dispensing device 4 is a prescription apparatus used for preparing a medicine. Specifically, as illustrated in Figs. 1, 2, and 3, the tablet dispensing device 4 includes a control unit 40, a tablet cassette 41, a manual dispensing unit 42, an individual dispensing unit 43, a rotating unit 44, a dispensing unit 45, a photographing unit 46, a passage detection unit 47, an operation display unit 48, a storage unit 49, and the like.

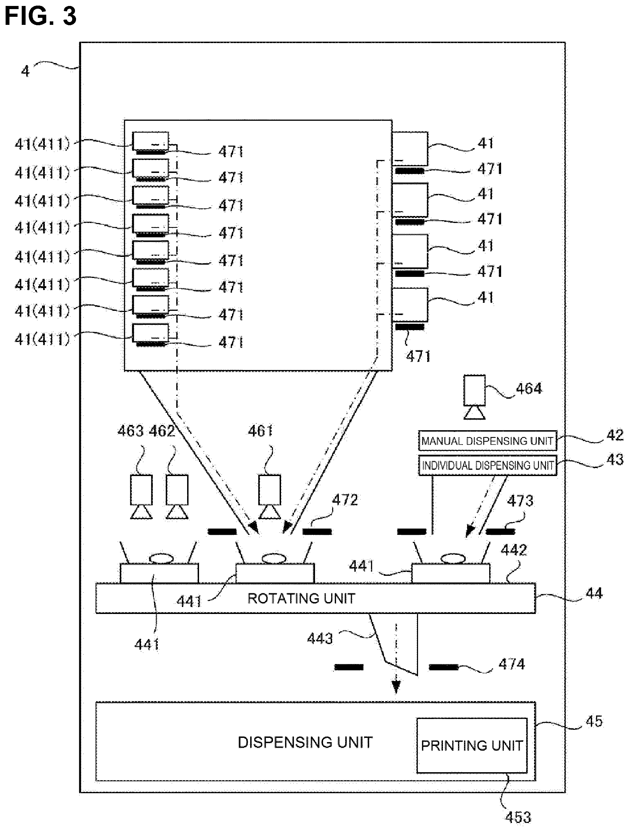

The tablet dispensing device 4 is capable of automatically dispensing tablets from any one or both of the tablet cassette 41 and the manual dispensing unit 42 on the basis of the prescription data to fold the tablets in units of dosage. In Fig. 3, alternate long and short dashed lines each indicate a moving path of a tablet.

The control unit 40 includes a processor such as a CPU, and a storage unit such as a RAM and an EEPROM, and performs various processes according to a dispensing control program stored in advance in the EEPROM, the storage unit 49, or the like, thereby comprehensively controlling the tablet dispensing device 4. Specifically, the control unit 40 controls operation of the tablet dispensing device 4 on the basis of the prescription data received from the server 2 so as to thereby cause the tablet dispensing device 4 to perform a dispensing process of folding one or more kinds of tablets corresponding to the prescription data for each timing of dosage. The dispensing control program is a program for causing the control unit 40 of the tablet dispensing device 4 to perform various processes, and is stored in advance in a recording medium such as a CD or a DVD. In the tablet dispensing device 4, the dispensing control program is read out from the recording medium by a drive unit (not shown), and is stored in the storage unit 49.

The operation display unit 48 includes a display unit such as a liquid crystal display, and an operation unit such as a touch panel. The storage unit 49 is a storage device, such as a hard disk drive or a solid state drive.

The tablet dispensing device 4 is provided with a plurality of tablet cassettes 41 that contains various tablets. In each of the tablet dispensing devices 4, a tablet listed in the prescription data as prescription medicine is automatically dispensed from the tablet cassette 41.

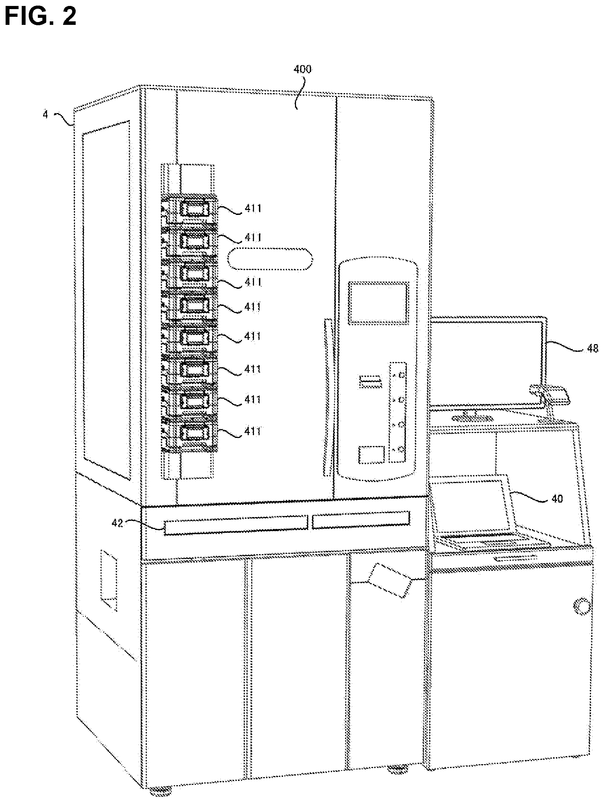

As illustrated in Fig. 2, the tablet cassettes 41 include one or more specific-tablet cassettes 411 detachable from the tablet dispensing device 4 when the front cover 400 of the tablet dispensing device 4 is in an open state and in an closed state. The specific-tablet cassette 411 is capable of dispensing any kinds of tablet in units of one tablet. For example, International Publication No. WO 2014/112221 discloses a cassette similar to the specific-tablet cassette 411.

The manual dispensing unit 42 is used for dispensing a tablet that is unsuitable for dispensing from the tablet cassette 41, such as a half-tablet and a quarter-tablet, having a size less than that of one tablet, and is provided in the tablet dispensing device 4 so as to be drawn from the tablet dispensing device 4. Specifically, the manual dispensing unit 42 includes a plurality of DTA squares 421 provided in a matrix (lattice shape), and a plurality of light emitting sections 422 provided corresponding to the plurality of DTA squares 421. For example, the light emitting section 422 is an LED capable of emitting light with an emission color selected from a plurality of preset emission colors by being controlled by the control unit 40. Each of the light emitting sections 422 is provided near the corresponding one of the DTA squares 421.

The individual dispensing unit 43 has a plurality of squares corresponding to the positions of the DTA squares 421 of the manual dispensing unit 42, and each of the squares of the individual dispensing units 43 is positioned under the corresponding one of the DTA squares 421 when the manual dispensing unit 42 is housed in the tablet dispensing device 4.

When the manual dispensing unit 42 is used, the manual dispensing unit 42 is drawn from a front face of the tablet dispensing device 4, and a tablet such as a half-tablet and a quarter-tablet, having a size less than that of one tablet, for example, is fed to each of the DTA squares 421. Subsequently, the tablet fed to each of the DTA squares 421 of the manual dispensing unit 42 is supplied to each of the squares of the individual dispending unit 43. For example, the manual dispensing unit 42 is provided such that a bottom surface of each of the DTA squares 421 is openable and closable. When the bottom surface is opened, the tablet fed to each of the DTA squares 421 falls to the corresponding one of the DTA squares 421 of the individual dispensing unit 43.

The individual dispensing unit 43 is capable of supplying the tablet contained in each of the squares of the individual dispensing unit 43 to the rotating unit 44 in units of square. As with the manual dispensing unit 42 and the individual dispensing unit 43, a manual dispensing unit capable of dispensing a tablet in units of square is disclosed in JP-A-2006-110386, for example.

For example, the individual dispensing unit 43 includes an opening-closing mechanism capable of sequentially opening and closing the bottom surface of each of the squares, and the bottom surface of each of the squares is sequentially opened by the opening-closing mechanism to sequentially dispense a tablet fed to each of the squares. More specifically, it is conceivable that the individual dispensing unit 43 supplies a tablet in each of the squares to the rotating unit 44 from each of the squares in a predetermined specific order.

Specifically, in the tablet dispensing device 4, the control unit 40 performs an allocating process of allocating each of tablets, which need to use the manual dispensing unit 42 in the dispensing process based on the prescription data, to the corresponding one of the DTA squares 421. For example, in the allocating process, each of the tablets is continuously allocated in the order along columns and rows where the DTA squares 421 are disposed. Fig. 23A is a schematic plan view of the manual dispensing unit 42, and Fig. 23B is a conceptual diagram illustrating an example of a result of an allocation to each of the DTA squares 421 of the manual dispensing unit 42.

As illustrated in Fig. 23A, in the manual dispensing unit 42, the DTA squares 421 includes eleven columns horizontally from the column A to the column K, six rows vertically from the first row to the sixth row for the column A to the column J, and three rows vertically from the first row to the third row for the column K. That is, the manual dispensing unit 42 includes 63 DTA squares 421 in total. In the following description, a position of the DTA square 421 may be specified by a combination of a column and a row of the manual dispensing unit 42, and for example, the DTA square 421 in the second row of the column A may be referred to as a DTA square 421A2.

Here, the prescription data to be subjected to the dispensing processes includes a tablet M1, a tablet M2, and a tablet M3 as prescription medicines that need to use the manual dispensing unit 42, and the tablets M1 and M3 each require three dosage times a day of in the morning, daytime, and evening, for two days, and a prescription dose of one tablet for each timing of dosage. In the present embodiment, morning, daytime, and evening are abbreviations for after breakfast, after lunch, and after dinner, respectively, and other examples of dosage time include before breakfast, before lunch, before dinner, between meals, before sleeping, and when necessary, for example. Meanwhile, the tablet M2 requires two dosage times a day of in the morning and evening, for two days, and a prescription dose of two tablets in the morning and that of one tablet in the evening. For example, in the prescription data, a total of four tablets of the tablet M1, the tablet M2, the tablet M2, and the tablet M3 are folded in the same medicine packet 451 for the morning of the first day.

In this case, as illustrated in Fig. 23B, the control unit 40 allocates the tablets corresponding to the timing of dosage to the DTA square 421A1, the DTA square 421A2, ..., in this order for each timing of dosage. In the present embodiment, "one tablet" is sometimes used as a term meaning a single tablet, and the "one tablet" includes a half-tablet, a quarter-tablet, and the like.

Specifically, as illustrated in Fig. 23B, the tablets M1, M2, M2, and M3 corresponding to the morning of the first day as the timing of dosage are allocated to the DTA square 421A1, the DTA square 421A2, the DTA square 421A3, and the DTA square 421A4, respectively. Likewise, the tablets M1, M2, M2, and M3 corresponding to the morning of the second day as the timing of dosage are allocated to the DTA square 421A5, the DTA square 421A6, the DTA square 421B1, and the DTA square 421B2, respectively. The tablets M1 and M3 corresponding to the daytime of the first day as the timing of dosage are allocated to the DTA square 421B3 and the DTA square 421B4, respectively, and the tablets M1 and M3 corresponding to the daytime of the second day as the timing of dosage are allocated to the DTA square 421B5, and the DTA square 421B6, respectively. In addition, the tablets M1, M2, and M3 corresponding to the evening of the first day as the timing of dosage are allocated to the DTA square 421C1, the DTA square 421C2, and the DTA square 421C3, respectively, and the tablets M1, M2, and M3 corresponding to the evening of the second day as the timing of dosage are allocated to the DTA square 421C4, the DTA square 421C5, and the DTA square 421C6, respectively. Each of the tablets may be sequentially allocated to the corresponding one of the DTA squares 421 in the order of a total of six timings of dosage in the morning, daytime, and evening of the first day, and in the morning, daytime, and evening of the second day.

When the manual dispensing unit 42 is used in the dispensing process based on the prescription data in the tablet dispensing device 4, the control unit 40 performs a manual dispensing support process of supporting a pharmacist to feed tablets to the manual dispensing unit 42 by guiding a position of each of the DTA squares 421 corresponding to one of the tablets to be fed to the manual dispensing unit 42 when the dispensing process is started. Specifically, in the manual dispensing support process, the control unit 40 causes the operation display unit 48 to display an inspection support screen D0 indicating the position of each of the DTA squares 421 corresponding to one of the tablets, and causes a position of each of the DTA squares 421 corresponding to one of the tablets to be displayed by using a lighting pattern of the light emitting section 422 corresponding to each of the DTA squares 421.

Fig. 24A illustrates an example of the inspection support screen D0, and Fig. 24B illustrates an example of a lighting pattern of the light emitting section 422. In the present embodiment, the light emitting section 422 is configured to emit light in six colors of preset emission colors L1 to L6.

First, in the manual dispensing support process, the control unit 40 allocates the emission color L1 to the tablet M1, allocates the emission color L2 to the tablet M2, and allocates the emission color L3 to the tablet M3. Then, the control unit 40 causes an arrangement display area A0 in which the emission colors L1 to L3 are respectively applied to the positions of the DTA squares 421 allocated to the tablets M1 to M3 to be displayed on the manual dispensing support screen D0. As a result, a user can easily grasp a feeding position of each of the tablets M1 to M3 to the corresponding one of the DTA squares 421 with reference to the manual dispensing support screen D0 to efficiently feed tablets to the manual dispensing unit 42.

The control unit 40 also causes a prescription display area B0 showing a list of the tablets M1 to M3 to be displayed on the manual dispensing support screen D0. In particular, the control unit 40 causes a background of each of the tablets M1 to M3 to be displayed in the same color as the corresponding one of the emission colors L1 to L3 corresponding to the tablets M1 to M3, respectively, in the prescription display area B0. This enables a user to easily grasp a color arrangement corresponding to the tablets M1 to M3.

Meanwhile, as illustrated in Fig. 24B, the control unit 40 causes the light emitting section 422 of each of the DTA squares 421 corresponding to one of the tablets M1 to M3 to light in a color arrangement of the emission colors L1 to L3 in the manual dispensing unit 42. As a result, a user can easily grasp a feeding position of each of the tablets M1 to M3 to the corresponding one of the DTA squares 421 with reference to the emission color of the light emitting section 422 to efficiently feed tablets to the manual dispensing unit 42.

On the manual dispensing support screen D0, an operation key K0 for switching display contents in the arrangement display area A0 and a lighting mode of the light emitting section 422 is also displayed. Then, in response to operation of the operation key K0, the control unit 40 switches the display contents of the arrangement display area A0 and the lighting pattern of the light emitting section 422 between a state where each of the tablets is individually displayed and a state where a plurality of tablets is simultaneously displayed.

Specifically, Figs. 25A and 25B illustrate a state where only the position of the DTA square 421 corresponding to the tablet M1 is indicated in the arrangement display area A0 and the light emitting section 422, Figs. 26A and 26B illustrate a state where only the position of the DTA square 421 corresponding to the tablet M2 is indicated in the arrangement display area A0 and the light emitting section 422, and Figs. 27A and 27B illustrate a state where only the position of the DTA square 421 corresponding to the tablet M3 is indicated in the arrangement display area A0 and the light emitting section 422. This enables a user to easily perform operation of feeding the tablets M1 to M3 to the DTA square 421 in order by operating the operation key K0.

As another embodiment, it is also conceivable that the control unit 40 can switch a plurality of coloring patterns preset by a predetermined operation on the operation display unit 48 or initial setting of the tablet dispensing device 4. More specifically, it is conceivable that the control unit 40 switches between a first color arrangement pattern in which a different color is displayed for each type of tablet as illustrated in Figs. 24A and 24B, and a second color arrangement pattern in which a different color is displayed for each dosage time (usage) as illustrated in Figs. 28A and 28B. Switching between the first color arrangement pattern and the second color arrangement pattern can also be performed while the manual dispensing support screen D0 is displayed.

In the second color arrangement pattern illustrated in Figs. 28A and 28B, the emission color L1 is allocated to the DTA square 421 corresponding to a tablet whose dosage time is morning, the emission color L2 is allocated to the DTA square 421 corresponding to a tablet whose dosage time is daytime, and the emission color L3 is allocated to the DTA square 421 corresponding to a tablet whose dosage time is evening. In this case, the control unit 40 also changes a display mode of the prescription data in the prescription display area B0 such that the number of tablets for each dosage time is displayed. The prescription display area B0 displays a six-digit number that corresponds to the tablets M1 to M3 such that each digit indicates the number of tablets corresponding to one of dosage times such as waking up, morning, afternoon, evening, before sleeping, and when necessary.

An allocation pattern of each tablet to the DTA square 421 is not limited to the one described here. Specifically, it is conceivable that a plurality of allocation patterns preset by a predetermined operation on the operation display unit 48 or initial setting of the tablet dispensing device 4 can be appropriately switched in the tablet dispensing device 4.

For example, the aforementioned Figs. 24A and 24B, and Figs. 28A and 28B correspond to a case of selecting a first allocation pattern for continuously allocating tablets corresponding to respective timings of dosage to the corresponding cells along a preset row or column.

Meanwhile, Figs. 29A and 29B correspond to a case of selecting a second allocation pattern for allocating a tablet corresponding to one of the prescribed timings of dosage to the plurality of cells belonging to a preset unit row or unit column. Here, the second allocation pattern is described as a pattern for allocating a tablet corresponding to one of timings of dosage to a plurality of DTA squares 421 for each column (an example of unit column), for example. Specifically, tablets packed in the same medicine packet 451 are allocated to the DTA squares 421 in the same column. That is, the tablets fed to a plurality of different DTA squares 421 in the same column are packed in the same medicine packet 451. In the second allocation pattern, the same type of tablets are allocated to the DTA squares 421 in the same row, and a blank is applied to a timing of dosage at which there is no same type of tablets. It is also conceivable that two columns of the DTA squares 421 are allocated for each of the timings of dosage.

When the second allocation pattern is selected, as illustrated in Figs. 29A and 29B, the control unit 40 allocates a total of six timings of dosage of the morning of the first day, the morning of the second day, the daytime of the first day, the daytime of the second day, the evening of the first day and the evening of the second day, respectively, to the column A, the column B, the column C, the column D, the column E, and the column F of the DTA squares 421 of the manual dispensing unit 42.

Specifically, the tablets M1, M2, M2, and M3 corresponding to the morning of the first day are allocated to the DTA square 421A1, the DTA square 421A2, the DTA square 421A3, and the DTA square 421A4 of the column A, respectively. The tablets M1, M2, M2, and M3 corresponding to the morning of the second day are allocated to the DTA square 421B1, the DTA square 421B2, the DTA square 421B3, and the DTA square 421B4 of the column B, respectively. Next, the tablets M1 and M3 corresponding to the daytime of the first day are allocated to the DTA squares 421C1 and DTA squares 421C4 of the column C, respectively, and the tablets M1 and M3 corresponding to the daytime of the second day are allocated to the DTA square 421D1 and the DTA square 421D4 of the column D, respectively. In addition, the tablets M1, M2, and M3 corresponding to the evening of the first day are allocated to the DTA square 421E1, the DTA square 421E2, and the DTA square 421E4 of the column E, respectively, and the tablets M1, M2, and M3 corresponding to the evening of the second day are allocated to the DTA square 421F1, the DTA square 421F2, and the DTA square 421F4 of the column F, respectively. A total of six timings of dosage of the morning, the daytime, and the evening of the first day, and the morning, the daytime, and the evening of the second day, may be allocated to the column A, the column B, the column C, the column D, the column E, and the column F of the DTA squares 421 of the manual dispensing unit 42, respectively, in the order listed above.

Even when the second allocation pattern is used, the control unit 40 can switch display contents in the arrangement display area A0 and each of the light emitting sections 422 between a state where the tablets M1 to M3 included in the prescription data as prescription medicines are individually displayed, and a state where the tablets M1 to M3 are displayed at the same time, in response to operation of the operation key K0, as with the first allocation pattern.

When the light emitting section 422 can express six emission colors, six types of tablets can be treated at most, however, seven or more types of tablets requiring the use of the manual dispensing unit 42 cannot be treated. In contrast, it is conceivable that the control unit 40 may treat seven or more types of tablets by combining emission colors of the light emitting section 422 and lighting modes, such as lighting and blinking, with each other, for example. It is also conceivable to allocate blinking of a predetermined specific emission color to the seventh and subsequent types of tablets exceeding six colors expressible by the light emitting section 422.

In addition, it is conceivable that the control unit 40 may switch tablets to be allocated to the corresponding emission colors L1 to L6 of the light emitting section 422, in response to the switching operation. Specifically, in an initial display state of the manual dispensing support screen D0 and the light emitting section 422, lighting modes of the emission colors L1 to L6 are allocated to the corresponding first to sixth types of tablets, and a blinking mode of the emission color L6 is allocated to the seventh and subsequent types of tablets. Next, when the switching operation is performed, the control unit 40 allocates the following: the lighting modes of the emission colors L1 to L6 to the corresponding seventh to twelfth types of tablets; a lighting-off mode to the first to sixth types of tablets; and a blinking mode of the emission color L6 to the thirteenth and subsequent types of tablets. Likewise, after that, when the switching operation is performed, the control unit 40 allocates the following: the lighting modes to the emission colors L1 to L6 to the corresponding thirteenth to eighteenth types of tablets; the lighting-off mode to the first to twelfth types of tablets; and the blinking mode of the emission color L6 to the nineteenth and subsequent types of tablets. Subsequently, when the switching operation is performed, the control unit 40 allocates the following: the lighting modes of the emission colors L1 to L6 to the corresponding nineteenth to twenty fourth types of tablets; the lighting-off mode to the first to eighteenth types of tablets; and the blinking mode of the emission color L6 to the twenty fifth and subsequent types of tablets. In addition, when the switching operation is performed, the control unit 40 allocates the following: the lighting mode of the emission color L1 to the twenty fifth types of tablets; the lighting-off mode to the first to twenty fourth types of tablets; and the blinking mode of the emission color L6 to the twenty sixth and subsequent types of tablets. As a result, the tablet dispensing device 4 can treat twenty five types of tablets at most.

In the dispensing process based on the prescription data, the number of columns of the DTA squares 421 of the manual dispensing unit 42 may be insufficient for the number of tablets requiring the use of the manual dispensing unit 42, or timings of dosage. In this case, it is conceivable that the control unit 40 divides the number of tablets requiring the use of the manual dispensing unit 42 or timings of dosage in the dispensing process based on the prescription data into multiple times within a range of enabling tablets to be fed into the manual dispensing unit 42 at one time, and allocates each of the tablets to the corresponding one of the DTA squares 421 for each of the divided times. Specifically, it is conceivable that the control unit 40 sequentially updates allocation of the number of each of the tablets, which can be allocated, to the corresponding DTA squares 421 every time the manual dispensing unit 42 is opened or closed.

In the tablet dispensing device 4, the control unit 40 can switch between presence and absence of the automatic inspection process in response to a user's operation or a control command from the server 2. For example, when the camera 461 or the like used in the automatic inspection process of the tablet dispensing device 4 fails, the automatic inspection process is automatically switched to invalid. The automatic inspection process takes time, so that the automatic inspection process may be set invalid depending on a user's will. In this way, when the automatic inspection process is not performed, it is unnecessary to dispense tablets one by one from each of the DTA squares 421. Then, it is conceivable that when the automatic inspection process is not performed, the control unit 40 allocates the tablets to each of the DTA squares 421 of the manual dispensing unit 42 in units of dosage. That is, when the automatic inspection process is not performed, each of the timings of dosage and each of the DTA squares 421 are allocated so that tablets per dosage are fed into each of the DTA squares 421. As a result, tablets in units of timing of dosage are supplied from each of the DTA squares 421 to the corresponding one of the tablet rotating units 441 of the rotating unit 44 via the individual dispensing unit 43, and then the tablets in units of timing of dosage are supplied to the dispensing unit 45, thereby reducing time required for the dispensing process. In this case, tablets on the tablet rotating unit 441 are photographed only once by the camera 462 or 463 while the tablet rotating unit 441 of the rotating unit 44 is not driven, for example.

As illustrated in FIG. 3, the photographing unit 46 includes cameras 461 to 464 provided in a moving path of tablets from the tablet cassette 41 to the dispensing unit 45, and in a moving path of tablets from the manual dispensing unit 42 to the dispensing unit 45. Images photographed by the cameras 461 to 464 are color or monochrome. The cameras 461 to 464 are used to photograph tablets for every tablet or every plural tablets before the tablets dispensed from the tablet cassette 41 or the manual dispensing unit 42 are folded with dispensing paper by the dispensing unit 45. Then, the control unit 40 causes the storage unit 49 to store the photographed images of the tablets taken by the cameras 461 to 464 while associating the images with the prescription data to be subjected to the dispensing process when the photographed images are taken, and transmits the photographed images to the server 2.

As illustrated in Fig. 3, the camera 461 is used to photograph the tablets supplied from the tablet cassette 41 to the rotating unit 44. The cameras 462 and 463 are used to photograph a plurality of different regions (e.g., front and back surfaces) in an outer periphery of a tablet rotating in a tablet rotating unit 441 described below, provided in the rotating unit 44. The camera 464 is used to photograph tablets stored in the manual dispensing unit 42.

In particular, when the dispensing process based on the prescription data is performed, the control unit 40 causes the camera 462 or 463 to take a plurality of images for each tablet, and causes the storage unit 49 to store the images while associating the images with the medicine packet 451 housing the tablets. More specifically, as described below, the control unit 40 performs a first photographing step of photographing the tablets using the camera 462 or 463 in a first dispensing process in which the dispensing process is performed first for the prescription data, and then performs a first storing step of storing the photographed image (first photographed image) of the photographed tablet while associating the image with the prescription data. In addition, the control unit 40 performs a second photographing step of photographing the tablets using the camera 462 or 463 in a second dispensing process that is the second and subsequent dispensing processes, and then performs a second storing step of storing the photographed image (second photographed image) of the photographed tablet while associating the image with the prescription data. Here, the control unit 40 at the time of performing the first storing step and the second storing step is an example of a first storage processing unit. Storing of the photographed image while associating it with the prescription data means that the control unit 21 or the control unit 40 causes the photographed images taken in each of the first dispensing process and the second dispensing process, corresponding to the prescription data, to be stored in a form of enabling each of the photographed images to be extracted. For example, each of the photographed images is stored while being associated with prescription identification information such as a prescription ID for identifying the prescription data. That is, the first photographed image taken in the first dispensing process and the second photographed image taken in the second dispensing process are stored while being indirectly associated with each other via the prescription identification information of the prescription data. The same applies to other information such as an inspection result stored while being associated with the prescription data as described below.

The control unit 40 also causes information allowing the image taken to be identified whether it is taken in the first dispensing process or the second dispensing process to be stored while being associated with each of the photographed images. In particular, the control unit 40 causes information indicating how many times the dispensing process for each prescription data is performed for each dispensing process performed based on the prescription data to be stored while associating the information with the photographed image taken in the dispensing process. For example, a photographed image taken in the first dispensing process based on the prescription data is stored while being associated with information indicating that the number of times of the dispensing process is the first time, a photographed image taken in the second dispensing process based on the prescription data is stored while being associated with information indicating that the number of times of the dispensing process is the second time, and a photographed image taken in the third dispensing process based on the prescription data is stored while being associated with information indicating that the number of times of the dispensing process is the third time. This enables the control unit 40 not only to determine whether each of the photographed images is the first photographed image taken in the first dispensing process performed for the prescription data, or the second photographed image taken in the second dispensing process, but also to determine the number of times of the dispensing process in which each of the photographed images is taken.

In addition, it is conceivable that after causing the storage unit 49 to store second photographed images of the medicine packet 451 taken in the second dispensing process performed at the second and subsequent dispensing processes for the prescription data, the control unit 40 deletes first photographed images or second photographed images corresponding to the medicine packet 451 taken in the first dispensing process and the second dispensing process performed prior to the dispensing process, from the storage unit 49. As a result, the control unit 40 causes only the photographed image taken by the latest dispensing process for each of the medicine packets 451 to be stored to delete unnecessary photographed images, and causes a recording medium like the storage unit 49 to preferentially store important information. In addition, it is conceivable that when deletion confirmation operation is performed by a user's operation or when automatic deletion setting is preliminarily set to be effective, the control unit 40 causes the previous first or second photographed images to be deleted. It is also conceivable that when the second photographed images taken in the second dispensing process for the prescription data are stored, the first photographed images taken in the dispensing process performed first are deleted, and when the second photographed images taken in the dispensing processes performed at third and subsequent dispensing processes for the prescription data are stored, the previous second photographed images are not deleted.

Meanwhile, the control unit 21 of the server 2 similarly receives the photographed images from the control unit 40, and causes the storage unit 22 to store the photographed images. Thus, it is conceivable that after receiving second photographed images of the medicine packet 451 taken in the second dispensing process performed at the second and subsequent dispensing processes for the prescription data and causing the storage unit 22 to store the second photographed images, the control unit 21 also deletes first photographed images or second photographed images corresponding to the medicine packet 451 taken in the first dispensing process and the second dispensing process performed prior to the dispensing process, from the storage unit 22. In addition, it is conceivable that when deletion confirmation operation is performed by a user's operation or when automatic deletion setting is preliminarily set to be effective, the control unit 21 causes the previous first or second photographed images to be deleted. It is also conceivable that when second photographed images taken in the second dispensing process for the prescription data are stored, the control unit 21 deletes first photographed image taken in the dispensing process performed first, and when second photographed images taken in the dispensing processes performed at third and subsequent dispensing process for the prescription data are stored, the control unit 21 does not delete the previous second photographed images.

In addition, the control unit 40 causes the storage unit 49 to store types of the tablets, the photographed images, and inspection results in the automatic inspection described below while associating them with the medicine packets 451 obtained in the dispensing process based on the prescription data. That is, the types of the tablets, the photographed images, and the inspection results are stored while being associated with each other for each timing of dosage included in the prescription data. Specifically, the control unit 40 causes each of inspection results for the corresponding one of the timings of dosage, of inspection results in the dispensing process based on the prescription data, to be stored while associating each of the inspection results with the corresponding one of the timings of dosage, in the automatic inspection process. The control unit 40 performing a storage process for storing each of the timings of dosage at the time when the photographed image is taken and each of the photographed images while associating them with each other, as described above, is also an example of the first storage processing unit. In addition, the control unit 40 performing a storage process of storing each of the inspection results of the automatic inspection process based on the photographed images corresponding to one of the timings of dosage and each of the timings of dosage while associating them with each other, is an example of a second storage process. Meanwhile, in the automatic inspection process, the control unit 40 also stores the inspection results of the whole of the dispensing processes based on the prescription data while associating the inspection results with the prescription data. It is also conceivable that the control unit 21 is configured to perform the automatic inspection process and store each inspection result and each of the timings of dosage while associating them with each other. In this case, the control unit 21 corresponds to a second storage processing unit.

As with the photographed images, the control unit 40 causes information allowing the inspection result to be identified whether it corresponds to the first dispensing process or the second dispensing process to be stored while associating the information with each of the inspection results. In particular, the control unit 40 causes information indicating how many times the dispensing process is performed for each dispensing process performed based on the prescription data to be stored while associating the information with the inspection result corresponding to the dispensing process. For example, an inspection result of the automatic inspection process performed in the first dispensing process based on the prescription data is stored while being associated with information indicating that the number of times of the dispensing process is the first time, an inspection result of the automatic inspection process performed in the second dispensing process based on the prescription data is stored while being associated with information indicating that the number of times of the dispensing process is the second time, and an inspection result of the automatic inspection process performed in the third dispensing process based on the prescription data is stored while being associated with information indicating that the number of times of the dispensing process is the third time. This enables the control unit 40 not only to determine whether each of the inspection results is the inspection result of the automatic inspection process corresponding to the first dispensing process or that corresponding to the second dispensing process, performed for the prescription data, but also to determine that each of the inspection results corresponds to how many times the dispensing process is performed.

It is also conceivable that the control unit 40 causes information indicating that how many times the dispensing process is performed for each of the timings of dosage, for each dispensing process performed for the corresponding one of the timings of dosage (medicine packet 451), and an inspection result of the automatic inspection process performed for each of the dispensing process for the corresponding one of the timings of dosage (medicine packet 451), to be stored while associating them with each other as history information. The control unit 40 at the time of performing such a storage process is an example of a storage processing unit. The history information may include the photographed images, or may not include the photographed images.

Then, the history information is stored while being associated with patient information, prescription data, etc., and is transmitted from the control unit 40 to the server 2. This enables the control unit 21 of the server 2 to extract arbitrary piece of the history information and cause the history information to be displayed, in response to a user's operation, for example. Such a display process is performed by the display processing unit 211. Here, each of Figs. 30A and 30B illustrates an example of the history information displayed by the display processing unit 211.

In the history information illustrated in Fig. 30A, information indicating how many times the dispensing process is performed for each of the medicine packets 451 is displayed, and an inspection result at each time of the dispensing processes is displayed for each medicine packet 451. Specifically, timings of dosage of the first to sixth packets are indicated as follows: in the first time, checking of each of timings of dosage of the first to fourth capsules indicates OK, and checking of each of timings of dosage of the fifth and sixth packets indicates NG; in the second time, checking of timing of dosage of the fifth packet indicates OK, and checking of timing of dosage of the sixth packet indicates NG; and in the third time, checking of timing of dosage of the sixth packet indicates OK. In addition, in the history information illustrated in Fig. 30B, information indicating how many times the dispensing process is performed is displayed for each dispensing process for each prescription data, and an inspection result for each dispensing process is displayed for each prescription data.

The passage detection unit 47 includes passage detection sensors 471 to 474, such as optical sensors for detecting passage of a tablet in a moving path of a tablet from the tablet cassette 41 to the dispensing unit 45, and in a moving path of a tablet from the manual dispensing unit 42 to the dispensing unit 45. Then, a detection signal of a tablet of each of the passage detection sensors 471 to 474 is input into the control unit 40.

As illustrated in Fig. 3, the passage detection sensor 471 detects a tablet dispensed from the tablet cassette 41, and the passage detection sensor 472 detects a tablet falling from the tablet cassette 41 to the rotating unit 44. The passage detection sensor 473 detects a tablet falling from the individual dispensing unit 43 to the rotating unit 44. The passage detection sensor 474 detects a tablet falling from the rotating unit 44 to the dispensing unit 45. Specifically, when the dispensing process based on the prescription data is performed, the control unit 40 causes the storage unit 49 to store the number of tablets detected by the passage detection sensor 474 in units of timing of dosage, as the number of dispensing tablets corresponding to each of the medicine packets 451.

The control unit 40 also performs a photographing process of photographing an image with the photographing unit 46 in response to a detection timing of a tablet performed by the passage detection unit 47, for example. Specifically, when a tablet falling from the tablet cassette 41 to the rotating unit 44 is detected by the passage detection unit 47, the tablet is photographed by the photographing unit 46. Photographing by the cameras 462 and 463 is performed at a predetermined photographing interval (several milliseconds) during performing of the dispensing process in the tablet dispensing device 4.

Meanwhile, in the tablet dispensing device 4, when an operation of inputting completion of a manual dispensing operation of tablets to the manual dispensing unit 42 is performed, the control unit 40 causes the camera 464 to take an image of the manual dispensing unit 42. When the manual dispensing unit 42 is configured to be drawn out of the tablet dispensing device 4 when being used, and to be housed in the tablet dispensing device 4 after a tablet is dispensed, it is also conceivable that the camera 464 is provided inside the tablet dispensing device 4, and the control unit 40 causes the camera 464 to take an image of the manual dispensing unit 42 at a timing when the manual dispensing unit 42 is housed in the tablet dispensing device 4. Then, the tablet dispensing device 4 transmits an image of manual dispensing taken by the camera 464 to the server 2 together with information for identifying the prescription data. As a result, in the server 2, the control unit 21 causes the storage unit 22 to store the image of manual dispensing while associating it with the prescription data. The dispensing management device 2 may be configured to read out the image of manual dispensing stored in the tablet dispensing device 4.

The control unit 40 is capable of performing an automatic inspection process of determining propriety of the dispensing process based on the prescription data, on the basis of a photographed image of a tablet taken by the camera 462 or 463 and the prescription data. Particularly, in the automatic inspection process, propriety of a dispensing process based on the prescription data is determined in units of the prescription data, and propriety of the dispensing process is also determined for each timing of dosage, on the basis of the photographed image of the tablet photographed by the camera 462 or 463 and the prescription data. The control unit 40 at the time of performing such an automatic inspection process is an example of an inspection processing unit. In the automatic inspection process, it is also conceivable as another embodiment that propriety of the dispensing process is determined in units of any one of the prescription data and the timing of dosage.

In the automatic inspection process, it is determined whether identification information on the tablet included in the photographed image of the tablet coincides with medicine information included in the prescription data. Specifically, an image of the tablet included in the photographed image and a correct image associated with a tablet included in the prescription data as prescription medicine may be checked with each other. The correct image is preliminarily registered while being associated with each of the types of tablets in the medicine master. In the automatic inspection process, identification information on the tablet is read out from the photographed image by pattern matching processing, character recognition processing, or the like, and the identification information on the tablet may be checked with identification information on a tablet included in the prescription data as prescription medicine.

It is also conceivable that the tablet dispensing device 4 is provided with a component detector for detecting medicine components before or after the dispensing process is performed, instead of or in addition to photographing means such as the cameras 462 and 463. The component detector is a qualitative analyzer capable of detecting components of the medicine on the basis of spectral characteristics of the medicine irradiated with light with a preset wavelength before or after the dispensing process is performed. Specifically, the qualitative analyzer is a device of infrared spectroscopy, powder X-ray analysis, mass spectrometry, Raman spectroscopy, or the like. In this case, in the automatic inspection process, the control unit 40 can identify the medicine on the basis of the medicine components detected by the component detector and check the medicine with the prescription data. The control unit 40 then causes the storage unit 49 to store spectrum waveforms or chemical components detected in the automatic inspection process as an inspection result while associating them with the prescription data or the like, as history information, and transmits the inspection result to the server 2.

When the checking result coincides in the dispensing process based on the prescription data, it is determined that the inspection result is appropriate, and when the checking result does not coincide, it is determined that the inspection result is an error. As described above, the control unit 40 is capable of determining whether the dispensing process is properly performed, on the basis of a photographed image taken by the camera 462 or 463 before a tablet is folded with the medicine packet 451 in the dispensing process and the prescription data. The control unit 40 determines whether the inspection result is appropriate in units of the prescription data and in units of the timing of dosage.

When performing the automatic inspection process using the identification information on the tablet acquired from the photographed image of the tablet, the control unit 40 transmits an original image when the identification information is read out from the tablet, among photographed images of the tablet taken by the camera 462 or 463, to the server 2 together with a result (coincidence or error) of the automatic inspection process. In the automatic inspection process, it is conceivable that the control unit 40 extracts the region of the tablet from the photographed image taken by the camera 462 or 463, and reads out identification information on the tablet on the basis of the extracted trimming image. In this case, the control unit 40 transmits the trimming image to the server 2 as the original image instead of or in addition to the photographed image.

When the tablet included in the prescription data as prescription medicine is a half-tablet or a quarter-tablet, having a size less than that of one tablet, the photographed image may not include a printed character or an engraved mark of the tablet, and thus it is difficult to perform the automatic inspection process on the basis of the photographed image. Thus, it is conceivable that when the tablet dispensed from the tablet cassette 41 or the manual dispensing unit 42 is a half-tablet or a quarter-tablet, having a size less than that of one tablet, the automatic inspection process based on the photographed image taken by the camera 462 or 463 is not performed in the tablet dispensing device 4. When the tablet included in the prescription data as prescription medicine is a half-tablet or a quarter-tablet, having a size less than that of one tablet, the control unit 40 processes an inspection result of the tablet as that to be checked.

As another embodiment, it is conceivable that even when the tablet has a size less than that of one tablet, the control unit 21 determines whether a color of the tablet included in the photographed image coincides with a color of the tablet registered in the medicinal master. In this case, it is conceivable that the control unit 21 processes an inspection result as that to be checked when the color of the tablet coincides, and processes the inspection result as an error when the color of the tablet does not coincide.

Even when the automatic inspection process is not performed, it is conceivable that when the tablet dispensed from the tablet cassette 41 or the manual dispensing unit 42 is a half-tablet or a quarter-tablet, having a size less than that of one tablet, the tablet placed on the tablet rotating unit 441 is photographed once by using any one of the cameras 462 and 463. At that time, the control unit 40 does not drive the tablet rotating unit 441, and the tablet placed on the tablet rotating unit 441 is photographed in a stationary state. The control unit 40 also causes the storage unit 49 to store a photographed image of a tablet having a size less than that of one tablet while associating the photographed image with the prescription data, and transmits the photographed image to the server 2.

Likewise, when the tablet included in the prescription data as prescription medicine is a predetermined medicine excluding an image inspection such as a transparent or translucent capsule tablet, a printed character or an engraved mark of the tablet may not clearly appear on the photographed image due to displacement of powder or liquid medicine contained in the capsule tablet. In this case, it is difficult to perform the automatic inspection process on the basis of the photographed image. Thus, it is conceivable that even when the tablet dispensed from the tablet cassette 41 or the manual dispensing unit 42 is a medicine excluding an image inspecting, the automatic inspection process based on a photographed image taken by the camera 462 or 463 is not performed in the tablet dispensing device 4. When the tablet included in the prescription data as prescription medicine is a half-tablet or a quarter-tablet, having a size less than that of one tablet, the control unit 40 processes an inspection result of the tablet as that to be checked.

In addition, when the tablet included in the prescription data as prescription medicine is an unregistered medicine whose correct image is not registered in the medicinal master, it is impossible to perform an inspection for propriety of the tablet based on the photographed images. Thus, it is conceivable that even when the tablet included in the prescription data to be subjected to the dispensing process is the unregistered medicine, an automatic inspection process based on photographed images taken by the camera 462 or 463 is not performed in the tablet dispensing device 4. When the tablet included in the prescription data as prescription medicine is the unregistered medicine, the control unit 40 processes an inspection result of the tablet as that to be checked.

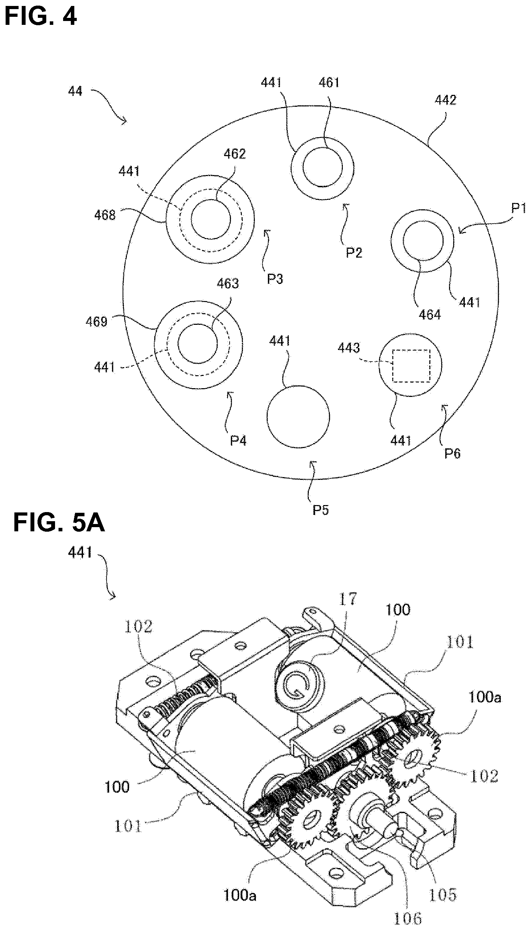

As illustrated in Figs. 3 and 4, the rotating unit 44 includes six tablet rotating units 441, a unit-rotating unit 442, and a medicine feeding unit 443. The unit-rotating unit 442 is rotatably supported by a base (not illustrated). Fig. 4 is a schematic diagram illustrating the rotating unit 44 as viewed from above.

Each of the tablet rotating units 441 can rotate one tablet supplied from the tablet cassette 41 or the manual dispensing unit 42 to change a posture of the tablet. The unit-rotating unit 442 is provided with the six tablet rotating units 441 disposed at intervals of 60° around a predetermined rotation axis, and the unit-rotating unit 442 can rotate each of the tablet rotating units 441 around the predetermined rotation axis. Specifically, the unit-rotating unit 442 moves each of the tablet rotating units 441 to the following six positions in order: a falling position P1 of a tablet from the individual dispensing unit 43; a falling position P2 of a tablet from the tablet cassette 41; a photographable position P3 by the camera 462; a photographable position P4 by the camera 463; a preliminary position P5; and a falling position P6 to the medicine feeding unit 443.

As illustrated in Fig. 5A, in the tablet dispensing device 4, when one tablet 17 dispensed from the tablet cassette 41 falls into the tablet rotating unit 441 or when one tablet 17 dispensed from the individual dispensing unit 43 falls into the tablet rotating unit 441, the tablet rotating unit 441 is rotated by 60° by the unit-rotating unit 442. As a result, each of tablets dispensed from the tablet cassette 41 or the manual dispensing unit 42 is sequentially moved from the falling position P1 or the falling position P2 toward the falling position P6 while being individually placed on the tablet rotating unit 441. Then, at the photographable position P3 and the photographable position P4, the tablet 17 is photographed by the camera 462 and the camera 463.

After that, the tablet 17 placed on the tablet rotating unit 441 falls from the falling position P6 to the medicine feeding unit 443. The medicine feeding unit 443 is used to temporarily store a tablet in units of dosage on the way of falling from the rotating unit 44 into the dispensing unit 45. Then, a bottom surface of the medicine feeding unit 443 is opened after a tablet in units of dosage is stored, and the tablet in units of dosage housed in the medicine feeding unit 443 is supplied to the dispensing unit 45.

As illustrated in Fig. 4, illumination devices 468 and 469 for illuminating the tablet at the photographable position by the cameras 462 and 463 are fixed above the rotating unit 44. The illumination devices 468 and 469 are configured to irradiate the tablet rotating unit 441 with light at angles or illuminances, different from each other, so that images under different lighting conditions are taken by the cameras 462 and 463.

For example, the illumination device 468 performs illumination suitable for reading the identification information on a tablet formed by being engraved on the tablet. For example, it is conceivable that the illumination device 468 irradiates the tablet with light from the side or diagonally above such that the identification information of the tablet is sharply photographed. The illumination device 468 may irradiate the tablet with light from a plurality of directions. This is because when the identification information on the tablet, which is formed on the tablet, is an engraved mark, the engraved mark causes irregularities on the surface of the tablet, and thus an appearance of the photographed identification information of the tablet may vary depending on an irradiation angle of the light on the irregularities.

In addition, the illumination device 469 performs illumination suitable for reading the identification information on a tablet formed by being printed on the tablet. For example, and it is conceivable that the illumination device 469 irradiates the tablet with light from above such that the identification information of the tablet is sharply photographed. This is because when the identification information on the tablet is formed on the tablet by printing, the identification information is formed on the surface of the tablet with paint or developer, and thus irregularities are not formed on the tablet, so that an appearance of the photographed identification information of the tablet is less likely to vary. Whether the identification information on the tablet formed on the tablet, such as a character and a reference sign, is formed on the tablet by being engraved or printed is registered in advance in medicine master or the like. The medicine master is stored not only in the storage unit 22 of the server 2 but also in the storage unit 49 provided in the tablet dispensing device 4.

For example, in the medicinal master, it is conceivable that an engraved mark flag is set to "1" for a tablet with the identification information engraved, and a print flag is set to "1" for a tablet with the identification information printed. In the medicine master, a tablet with the identification information engraved has an engraved mark flag set to "1", and a tablet with the identification information printed may have an engraved mark flag set to "0". Then, the tablet dispensing device 4 uses a photographed image suitable for a method (engraving or printing) for forming the identification information of the tablet, among photographed images taken by the cameras 462 and 463, for the automatic inspection process.

As with the server 2, the storage unit 49 stores a medicine database in which information such as a medicine code, a medicine name, a JAN code, an RSS code, a medicine bottle code, a dosage form, a unit, specific gravity, a medicine type, a blending variation, a pharmaceutical excipient, precautions, allergy information, information in attached documents and the like are associated with each other for each medicine, separately from the medicine master. Particularly, the medicine database stores information such as identification information on a tablet, formed on the tablet, a shape of the tablet, appearance images of the tablet (front and back surfaces) and the like, for each tablet. The medicine database is read out from a recording medium such as a CD or a DVD by a drive unit (not illustrated) provided in the tablet dispensing device, or is received from an external device like the server 2 via the communication network N1, to be stored in the storage unit 49. In addition, the control unit 40 may be configured to read out the medicine database from an external device like the server 2, or from a website via the communication network N1 as required. The medicine database is used for updating the medicine master, for example.

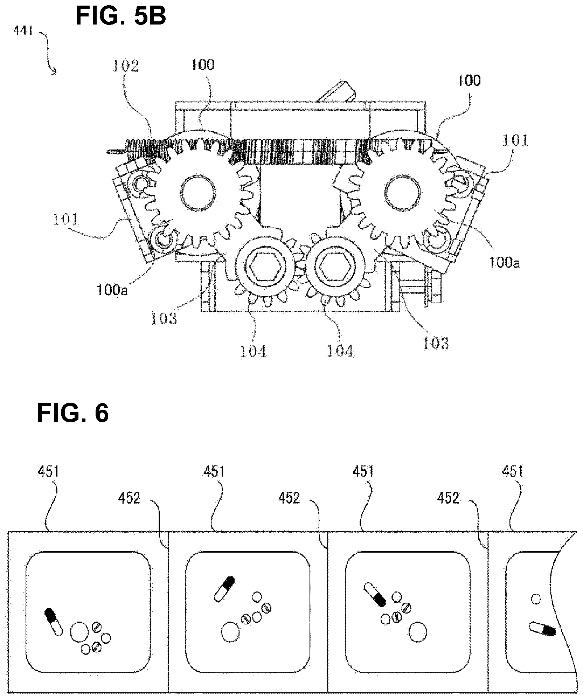

Here, an example of the tablet rotating unit 441 will be described with reference to Figs. 5A and 5B. As illustrated in Figs. 5A and 5B, the tablet rotating unit 441 includes a pair of rotating rollers 100, a pair of supporting plates 101 that individually support the corresponding rotating rollers 100, and a spring 102 for urging the pair of supporting plates 101 in a direction to approach each other. The tablet rotating unit 441 includes arms 103 that extend from both respective end portions of the supporting plate 101, and that are provided at their leading end portion with respective gears 104 meshing with each other. As a result, under normal conditions, the rotating rollers 100 are close to each other, and the tablet 17 can be supported by the pair of rotating rollers 100. Meanwhile, the pair of rotating rollers 100 is brought into contact with and separated from each other synchronously with rotation of the gears 104. The pair of rotating rollers 100 rotatably supports the medicine 17 while being in contact with or close to each other, and allows the medicine 17 to fall from the pair of rotating rollers 100 toward the medicine feeding unit 443 while being separated from each other.

One end portion of each of the rotating rollers 100 is integrally provided with a driven gear 100a. Each of the driven gears 100a meshes with a drive gear 106 provided integrally with one end of a drive shaft 105. The other end side of the drive shaft 105 is integrally provided with a driven roller (not illustrated). The driven roller is composed of a magnet gear. The rotating unit 44 is provided with a coupling magnet gear (not illustrated) connected to a predetermined driving motor, at a position just under and away from the tablet rotating unit 441, when the tablet rotating unit 441 is moved to the photographable position P3 by the camera 462 or the photographable position P4 by the camera 463.

When the tablet rotating unit 441 is moved to the photographable position P3 of the camera 462 or the photographable position P4 of the camera 463, a driving force from the predetermined driving motor is transmitted to the driven roller via the coupling magnet gear. As a result, the driving force of the predetermined driving motor is transmitted to the pair of rotating rollers 100 via the driven roller and the drive shaft 105 to synchronously rotate the pair of rotating rollers 100 in the same direction. Thus, when the tablet 17 is placed on the pair of rotating rollers 100, the tablet 17 is rotated. As a result, the tablet rotating unit 441 enables a posture of the tablet 17 to be photographed by the cameras 462 and 463 to be changed, so that an outer peripheral surface of the tablet 17 can be photographed from different directions. That is, when the tablet 17 rotated by the tablet rotating unit 441 is intermittently or continuously photographed by the cameras 462 and 463, the outer peripheral surface of the tablet 17, including identification information formed by an engraved mark or the like, can be photographed.

The structure of the tablet rotating unit 441 described above is merely an example, and the tablet rotating unit 441 may have any structure as long as a tablet can be displaced such that the cameras 462 and 463 or the like can take an image from which identification information on the tablet can be read out. Then, it is desirable that the tablet is photographed such that the identification information of the tablet clearly appears in the photographed image. As long as the outer peripheral surface of the tablet can be photographed from different directions, the tablet dispensing device 4 may be configured such that the tablet is placed on a transparent mounting plate to allow the tablet to be photographed from front and back surfaces of the mounting plate, for example.

The dispensing unit 45 is capable of folding a tablet in units of dosage supplied from any one of or both of the tablet cassette 41 and the manual dispensing unit 42 via the rotating unit 44 with a medicine packet 451. Here, Fig. 6 illustrates an example of the medicine packet 451 dispensed from the tablet dispensing device 4. As illustrated in Fig. 6, each of the medicine packets 451 is packed with a plurality of tablets in units of dosage, and a tear-off dotted line (perforation) 452 for easily separating each of the medicine packets 451 is formed between each of the medicine packets 451.

The dispensing unit 45 is provided with a printing unit 453 for printing information on each of the medicine packets 451, so that information such as the name of a patient, a dosage timing (or dosage time), a prescription medicine, a prescription amount, or the like can be printed on a surface of each of the medicine packets 451 by the printing unit 453. In addition, the dispensing unit 45 attaches an empty medicine packet 451, on which one-dimensional or two-dimensional code information indicating prescription identification information such as a prescription ID for identifying the prescription data is printed, to the first or the last of a series of the medicine packets 451 obtained by the dispensing process based on one prescription data. The code information can be read out by the code reading unit 27 and the code reading unit 37. The code information may be printed on each of the medicine packets 451.

### [Prescription Apparatus 5]

As with the tablet dispensing device 4, the prescription apparatus 5 is used for dispensing a medicine on the basis of prescription data. The prescription apparatus 5 includes a powder medicine dispensing device, a liquid medicine dispensing device, a sheet dispensing device, a picking supporting device, and the like, in addition to the tablet dispensing device 4, for example. The powder medicine dispensing device includes a plurality of powder medicine cassettes housing a plurality of kinds of powdered medicine, and is capable of automatically folding powdered medicine housed in the powdered medicine cassette by a predetermined amount according to the prescription data. The liquid medicine dispensing device includes a plurality of medicine bottles containing a plurality of types of liquid medicine, and dispenses a requirement of liquid medicine from one of the medicine bottles according to prescription data. The sheet dispensing device dispenses a PTP sheet or a heat-sealing sheet with which a tablet is packed in advance from one of a plurality of sheet cassettes in which the PTP sheet or the heat seal is housed according to the prescription data. The picking supporting device is used to read out a medicine name from identification information (barcode or the like) attached to a medicine shelf or a medicine bottle to check the read out medicine name with a medicine name included in prescription data when a pharmacist manually prepares a medicine.

In a medical facility such as a hospital or a pharmacy, a pharmacist conducts an inspection operation for checking whether tablets folded by the tablet dispensing device 4 are appropriate for prescription data. In contrast, the inspection support system 1 supports an inspection operation by a pharmacist by performing an inspection support process (refer to Fig. 7) described below.

### [Inspection Support Process]

Hereinafter, with reference to Fig. 7, an example of a procedure of an inspection support process performed by the control unit 21 of the server 2 in the inspection support system 1 will be described. The inspection support process is performed when a login operation to the server 2 is performed to the client terminal 3 by a pharmacist having a preset final inspection authority or when an inspection starting operation for performing a final inspection process is performed after the login operation, for example. The login authentication to the server 2 is performed on the basis of the user master stored in the storage unit 22. "Display" and "operation" described below are performed using the display unit 33 and the operation unit 34 of the client terminal 3 on which the login operation is performed. As a matter of course, similar operation and display may be performed by the server 2.

### <Step S11>

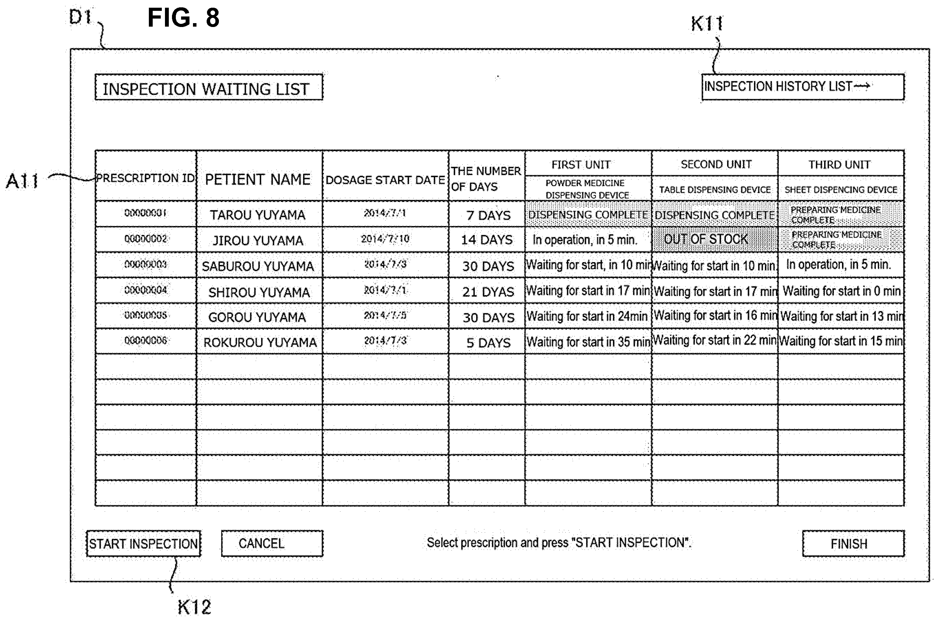

First, in step S11, the control unit 21 causes the client terminal 3 to display an inspection waiting list screen D1 for displaying a list of prescription data that is a candidate for an inspection object. Here, Fig. 8 illustrates an example of the inspection waiting list screen D1.

As illustrated in Fig. 8, the inspection waiting list screen D1 displays a list displaying area A11 that displays a list of prescription data to be inspected. Specifically, the list displaying area A11 shows information on a prescription ID (prescription identification information), a patient name (patient identification information), a dosage start date, and the number of days, included in prescription data, together with status information of a plurality of dispensing devices such as the tablet dispensing device 4 and the prescription apparatus 5, connected to the inspection support system 1.

Here, as illustrated in Fig. 8, the powder medicine dispensing device and the sheet dispensing device of the prescription apparatus 5 are registered in advance as a first unit and a third unit, respectively, and the tablet dispensing device 4 is registered in advance as a second unit. The status information is classified in advance into "dispensing complete", "preparing medicine complete", "during dispensing", "in operation", "waiting for start", "out of stock", and the like. The "dispensing complete" and "preparing medicine complete" indicate that dispensing of a medicine is completed, and a background or characters are displayed in a predetermined first specific color such as blue. The "during dispensing" and "in operation" indicate that dispensing of a medicine is being performed, and are displayed together with a waiting time until completion. The "waiting for start" is a standby state until dispensing of a medicine starts, and is displayed together with the waiting time until start. The "out of stock" indicates that dispensing cannot be started because a medicine, the medicine packet 451, or consumables such as an ink ribbon, which is used in dispensing based on prescription data, is in a stockout state, and a background or characters are displayed in a predetermined second specific color such as red.

In addition, the inspection waiting list screen D1 displays operation keys K11 to K12 and the like for receiving a user's operation. The operation key K11 is an operation key for displaying an inspection history screen D2 for displaying inspection history in the server 2, and the operation key K12 is an operation unit for displaying an inspection screen D3 for performing an inspection process in the server 2. That is, the control unit 21 can start an inspection of dispensing performed by a plurality of devices such as the tablet dispensing device 4 and the prescription apparatus 5 in response to an operation on the inspection waiting list screen D1.

### <Step S12>

In step S12, the control unit 21 determines whether a display operation in the inspection history screen D2 is performed. Specifically, when the operation key K11 on the inspection waiting list screen D1 is operated, the control unit 21 determines that a display operation on the inspection history screen D2 is performed. When determining that the display operation on the inspection history screen D2 is performed (Yes at S12), the control unit 21 shifts processing to step S13, and when the display operation on the inspection history screen D2 is not performed (No at S12), the control unit 21 shifts the processing to step S14.

### <Step S13>

In step S13, the control unit 21 causes the client terminal 3 to display the inspection history screen D2. In this way, the control unit 21 enables displaying of inspecting history of dispensing performed by a plurality of dispensing devices such as the tablet dispensing device 4 and the prescription apparatus 5, in response to a user's operation on the inspection waiting list screen D1.

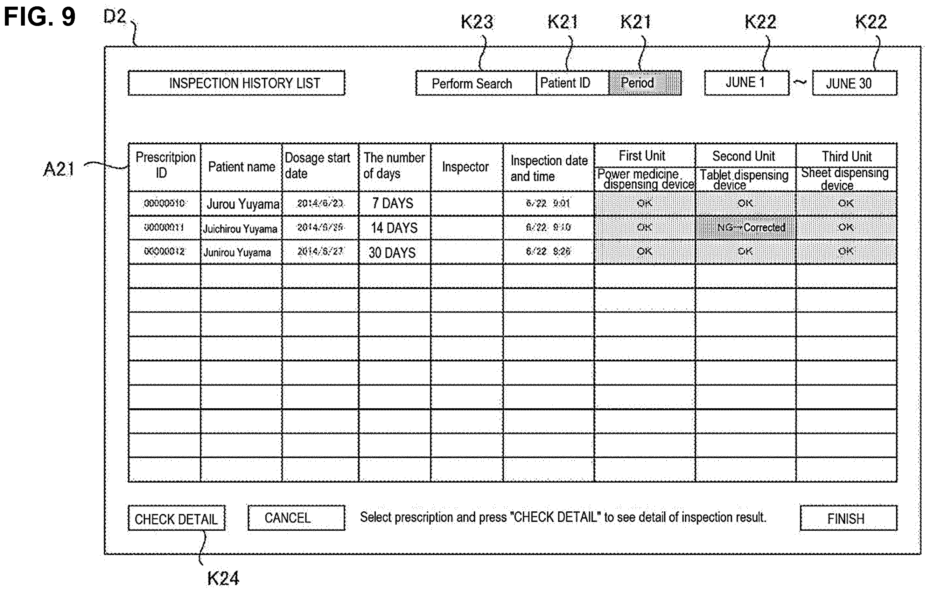

Fig. 9 illustrates an example of the inspection history screen D2. As illustrated in Fig. 9, the inspection history screen D2 displays a list displaying area A21 on which a list of prescription data that has been already inspected is displayed. In the list displaying area A 21, information such as a prescription ID (prescription identification information), a patient name (patient identification information), a dosage start date, an inspector, and an inspection date and time, included in prescription data, are displayed. In addition, "OK" indicating that a determination result is appropriate and "NG" indicating that a determination result is an error are displayed on the list displaying area A21, for example, as a result of inspection of medicines dispensed by each of the tablet dispensing device 4 and the prescription apparatus 5 connected to the inspection support system 1. Specifically, an inspection result corresponding to the tablet dispensing device 4 is a result of the automatic inspection process. The "OK" is displayed such that its background or characters are displayed in a predetermined third specific color such as blue or the like, and the "NG" is displayed such that its background or characters are displayed in a predetermined fourth specific color such as red or the like. When the "NG" is displayed, contents of treatment (e.g., "corrected") performed in response to a determination result being an error are displayed.

The inspection history screen D2 also displays an operation key K21 for selecting any one of a "patient ID" and a "period" as a search item, an input field K22 used for inputting search contents of the item selected by the operation key K21, an operation key K23 for performing a search, and the like. When search contents are input into the input field K22 and the operation key for "performing a search" is operated, the control unit 21 searches prescription data corresponding to the search contents and causes the prescription data to be displayed in the list displaying area A21. The inspection history screen D2 displays an operation key K24 for displaying an inspection screen similar to an inspection screen D3 described below that displays details of the inspection history of the prescription data displayed in the list displaying area A21.

### <Step S14>

In step S14, the control unit 21 determines whether the inspection starting operation is performed. Specifically, when the operation key K12 is operated while the prescription data to be inspected is selected on the inspection waiting list screen D1, the control unit 21 determines that the inspection starting operation is performed. When determining that the inspection starting operation is performed (Yes at S14), the control unit 21 shifts processing to step S15, and when the inspection starting operation is not performed (No at S14), the control unit 21 shifts the processing to step S16. Even when code information described in the medicine packet 451 is read out by the code reading unit 27 to identify the prescription data on the basis of the code information, for example, the control unit 21 determines that the inspection starting operation for the prescription data to be inspected is performed.

### <Step S15>

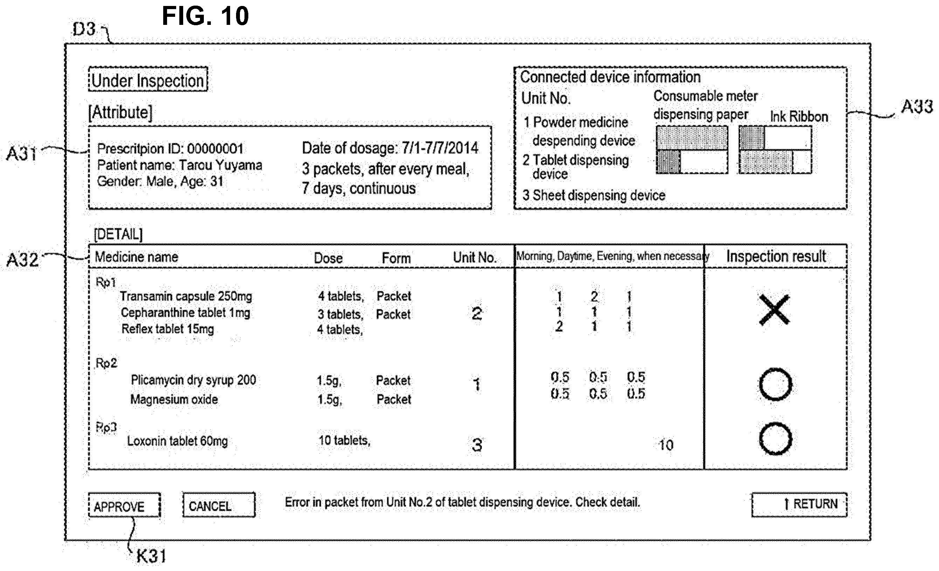

In step S15, the control unit 21 causes the client terminal 3 to display the inspection screen D3 for performing an inspection on the prescription data selected on the inspection waiting list screen D1. Fig. 10 illustrates an example of the inspection screen D3. As illustrated in Fig. 10, the inspection screen D3 displays a basic information area A31, an inspection result area A32, and a device information area A33. The basic information area A31 displays information such as a prescription ID (prescription identification information), a patient name (patient identification information), a gender, an age, a date of dosage, usage, and the like, included in prescription data.

In the inspection result area A32, contents of the prescription data selected on the inspection waiting list screen D1 and an inspection result of the prescription data are displayed for each of records (Rp1 to Rp3). Specifically, in the inspection result area A32, there are displayed a medicine name, a dose, a form, a unit number of a dispensing device, a dose, and a determination result. The determination result is obtained by determining propriety in an automatic inspection process performed in each of the tablet dispensing device 4 and the prescription apparatus 5, such as in the automatic inspection process performed in the tablet dispensing device 4, and is appropriately input into the server 2 from each of the tablet dispensing device 4 and the prescription apparatus 5.

The automatic inspection process may be performed by the control unit 21 of the server 2 that acquires various kinds of information such as images from the tablet dispensing device 4 and the prescription apparatus 5. Specifically, in the server 2, the control unit 21 causes the storage unit 22 to store an inspection result of propriety of a prescription process performed by each of the tablet dispensing device 4 and the prescription apparatus 5, and prescription identification information such as a prescription ID of the prescription data, while associating them with each other.

Then, the control unit 21 enables the inspection result of each of the tablet dispensing device 4 and the prescription apparatus 5 associated with the prescription data of the prescription data to be collectively displayed on one screen like the inspection result areas A32 in the inspection screen D3. This enables a user to collectively find out the inspection results of a plurality of the prescription apparatuses 5 including the tablet dispensing device 4 and the like that perform dispensing on the basis of the prescription data. For example, it is conceivable that the inspection screen D3 illustrated in Fig. 10 shows Rp1 that indicates a medicine dispensed by the tablet dispensing device 4, Rp2 that indicates a medicine dispensed by the powder medicine dispensing device of the prescription apparatuses 5, and Rp3 that indicates a medicine dispensed by the sheet dispensing device of the prescription apparatuses 5.

The device information area A33 displays remaining amount information on consumables in the tablet dispensing device 4 and the prescription apparatus 5, connected to the server 2. The remaining amount information on consumables displayed in the device information area A33 is appropriately input to the server 2 from each of the tablet dispensing device 4 and the prescription apparatus 5, or is read out from the tablet dispensing device 4 and the prescription apparatus 5 by the server 2. This enables a user to easily find out a remaining amount of consumables in the tablet dispensing device 4 and the prescription apparatus 5. For example, in the example illustrated in Fig. 10, information on consumables of the tablet of the "second unit" being the dispensing device 4 shows a state where a remaining amount of "dispensing paper" is small and a remaining amount of "ink ribbon" is sufficient. Likewise, information on consumables of the "first unit" being the powder medicine dispensing device shows a state where a remaining amount of "dispensing paper" is sufficient and a remaining amount of "ink ribbon" is small. In this way, the control unit 21 enables information on consumables in a plurality of dispensing devices such as the tablet dispensing device 4 and the prescription apparatus 5 to be displayed in the inspection screen D3 as a list. Thus, a user can easily manage consumables of the entire inspection support system 1. This enables the consumables to be prepared without omission, so that even a small number of users can continuously operate a plurality of dispensing devices such as the tablet dispensing device 4 and the prescription apparatus 5.

### <Step S16>

In step S16, the control unit 21 determines whether a detailed checking operation for checking details of a determination result displayed on the inspection screen D3 is performed. Specifically, it is determined that the detailed checking operation is performed when a display location of a determination result corresponding to any of the records included in the prescription data displayed in the inspection screen D3 is selected on the inspection screen D3. When determining that the detailed checking operation is performed (Yes at S16), the control unit 21 shifts processing to step S17, and shifts the processing to step S18 when the detailed checking operation is not performed (No at S16).

### <Step S17>

In step S17, the control unit 21 performs an inspection display control process for displaying an inspection detail screen D303 that displays details of a determination result displayed on the inspection screen D3. The screening display control process is performed by the display processing unit 211 of the control unit 21.

### [Inspection Display Control Process]

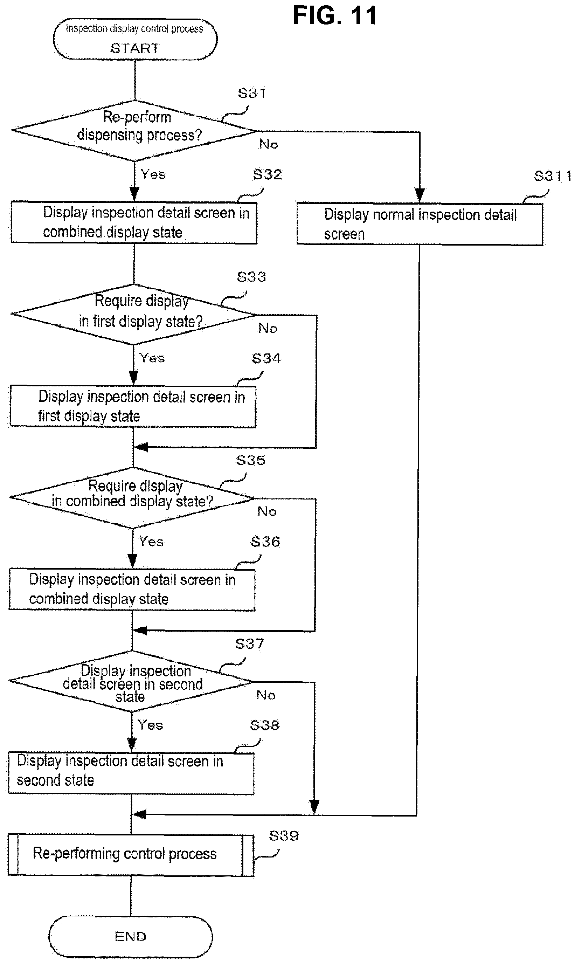

Hereinafter, an example of the inspection display control process performed by the control unit 21 will be described with reference to Fig. 11.

### <Step S31>

In step S31, the control unit 21 determines whether a part or all of the dispensing process is re-performed for the prescription data of the current inspection object.
Specifically, when re-performing of part or all of the dispensing processesing for the same prescription data is performed, the control unit 21 causes an inspection result of the dispensing processes re-performed to be stored while associating the inspection result with the prescription data. For example, the control unit 21 causes an inspection result during performing of the first dispensing process, an inspection result during re-performing of the first dispensing process, and an inspection result during re-performing of the second dispensing process, to be stored while associating the inspection results with the prescription data. A text, "inspection results are stored while being associated with the prescription data", means that the inspection results are stored while being associated with prescription identification information such as a prescription ID for identifying the prescription data, for example. When the inspection result during re-performing of the dispensing process is stored while being associated with the prescription data, the control unit 21 determines that the dispensing process is re-performed for the prescription data. When a part or all of the prescription process is re-performed for the same prescription data, it may be determined whether the dispensing process is re-performed by setting a re-performing flag corresponding to the prescription data to "ON" and referring to the re-performing flag. When it is determined that the re-performing is performed for the prescription data (Yes at S31), processing proceeds to step S32, and when it is determined that the re-performing is not performed (No at S31), the processing proceeds to step S311.

Hereinafter, for the convenience of explanation, the first dispensing process performed for the prescription data is referred to as a first dispensing process, and the second and subsequent dispensing processes are referred to as a second dispensing process in some cases. In the following description, there is first described processing when it is determined that the re-performing for the prescription data is not performed in step S31 (No at S31) in the inspection display control process, and processing when it is determined that the re-performing for the prescription data is performed in step S31 (Yes at S31) will be described later.

### <Step S311>

In step S311, the control unit 21 causes an inspection detail screen D303 to be displayed, the inspection detail screen D303 displaying an inspection result of the automatic inspection process performed for the first dispensing process performed for the prescription data for the current inspection object. Specifically, the control unit 21 causes the inspection result to be displayed on the inspecting detail screen D303 together with photographed images of a tablet taken in the first dispensing process.

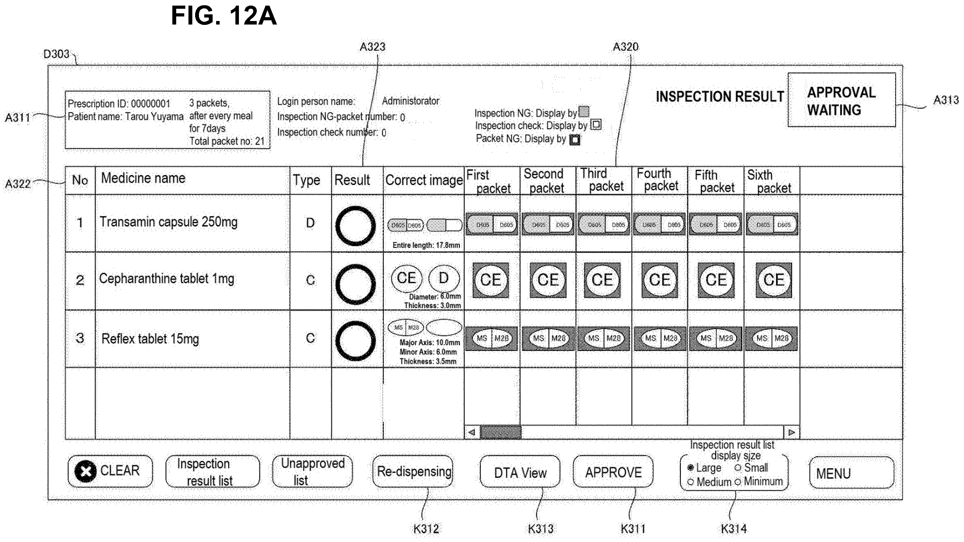

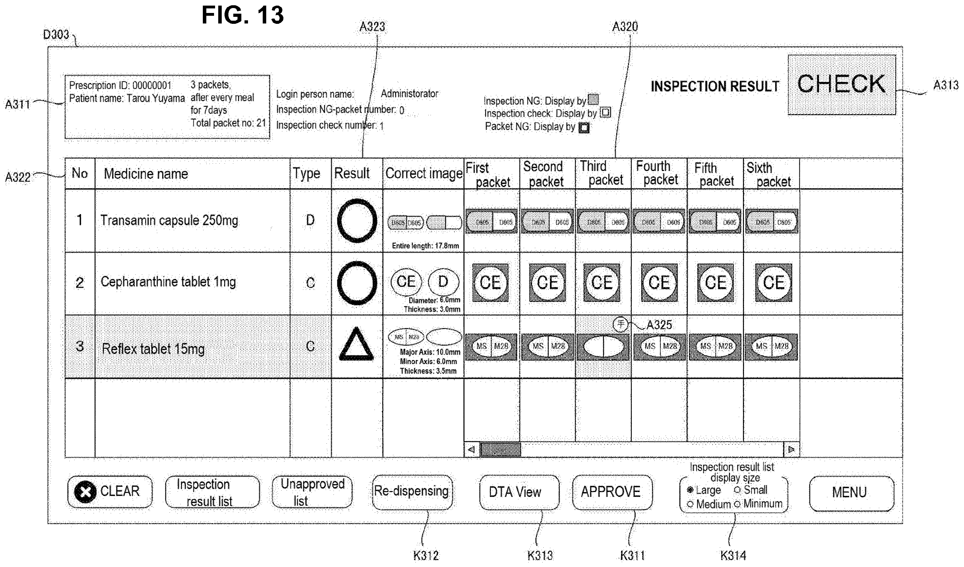

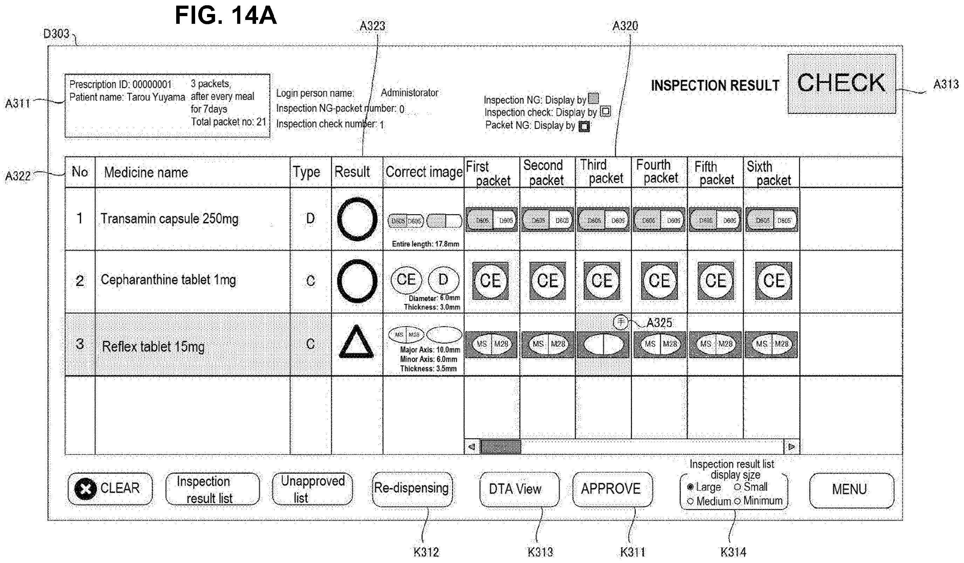

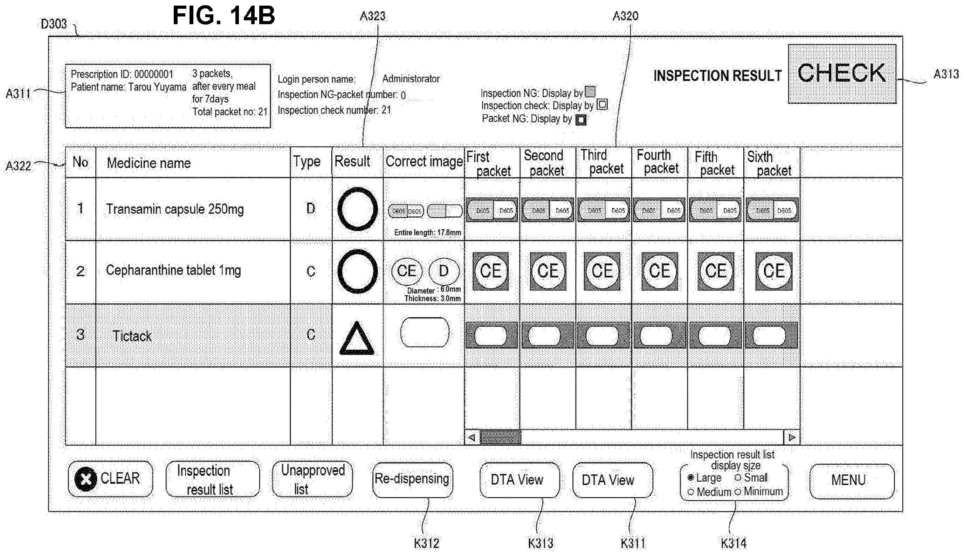

Figs. 12A, 13, and 14A each illustrate the inspection detail screen D303. Specifically, Fig. 12A shows a display example when an inspection result by the automatic inspection process is appropriate, Fig. 13 shows a display example when an inspection result by the automatic inspection process is an error, and Fig. 14 shows a display example when an inspection result by the automatic inspection process needs to be checked.

The inspecting detail screen D303 displays a basic information area A311, a dispensing detail area A 322, and an inspection result area A 313. As with the basic information area A31, the basic information area A311 is an area in which patient information, prescription data, and the like are displayed. More specifically, the basic information area A31 displays prescription-related information such as a prescription ID, a patient name, and usage, in the prescription data. The basic information area A31 may display other information such as a dose in the prescription data as the prescription related information. In the dispensing detail area A322, contents of a tablet folded with the medicine packet 451 are displayed in units of the medicine packet 451 (in units of dosage timing) side by side in a predetermined direction.

In the dispensing detail area A322, it is also conceivable that the display area A320, in which number-of-packets information indicating the number of packets of the medicine packet 451 such as the first packet, the second packet, etc. is displayed, displays a timing of dosage (a dosage date and a dosage time) together with or in place of the number-of-packets information. In addition, it is also considered that the control unit 21 causes a plurality of photographed images (e.g., a front surface image and a back surface image) of the tablet to be alternately switched and displayed in the dispensing detail area A322 in response to a selection operation for the photographed images of the tablet. This enables states of the tablet viewed from a plurality of directions to be found out while a display area in the normal state is reduced in size.

In the dispensing detail area A322 illustrated in Fig. 12A, a medicine name, a type, a result, and a correct image are displayed. Specifically, for the type, it is distinguishably displayed from which of the tablet cassette 41 and the manual dispensing unit 42 a tablet corresponding to the medicine name is dispensed. For example, "C", "K", or the like is displayed as an identification code when a dispensing source of the tablet is the tablet cassette 41, and "D", "Hand", or the like is displayed as an identification code when a dispensing source of the tablet is the manual dispensing unit (detachable tablet adapter) 45. For the result, propriety of an inspection result of a tablet corresponding to the medicine name is individually displayed. The correct image is a registered image that is preliminarily registered in the pharmaceutical master or the like as a correct image of the tablet. For example, in the medicine master, two images corresponding to the front and back surfaces of the tablet are registered as the registered images while being associated with the corresponding tablet. The registered image may be a single image obtained by photographing an area including identification information on the tablet in an outer peripheral surface of the tablet.

In the dispensing detail area A322, photographed images of a tablet taken by the photographing unit 46 before being folded in each of the medicine packets 451 are displayed as dispensing result information while being associated with the corresponding tablet. The photographed image displayed in the dispensing detail area A322 is an example of dispensing result information. As described above, when the tablet dispensing device 4 includes the component detector, a detection result detected by the component detector, such as a detected component and a medicine name, may be displayed as dispensing result information, in place of the photographed images. It is conceivable, as another embodiment, that a display of the photographed image and the detection result is eliminated, and any one of or a plurality of inspection results of the automatic inspection process for each prescription data, for each medicine, or for each timing of dosage, is displayed as dispensing result information. It is also conceivable, as another embodiment, that a display of inspection results of the automatic inspection process for each prescription data, for each medicine, or for each timing of dosage, is eliminated, and only the photographed image or the detection result is displayed as dispensing result information.

In a placement form in which tablet names (medicine names) are displayed in one of a column or a row and the a dispensing order (the first packet, the second packet,...) is displayed in the other, the control unit 21 causes each of photographed images of the tablets corresponding to the one of combinations of the tablet names and the dispensing orders to be displayed. In the dispensing detail area A322 illustrated in Fig. 12A, tablet names are vertically displayed side by side, and dispensing orders are laterally displayed. As described above, photographed images of the same type of tablet are placed in a direction of placement of the dispensing orders, so that a pharmacist can easily find out propriety of each of the photographed images of the corresponding one of the tablets.

In addition, the control unit 21 causes a plurality of photographed images to be displayed for each of the tablets, the plurality of photographed images including at least an original image from which the identification information of the tablet is read out by the tablet dispensing device 4 or an original image compared with the correct image, and corresponding to respective different outer peripheral surfaces of the tablet photographed in the tablet dispensing device 4A for each of the tablets. This enables a pharmacist to determine propriety of the tablet by referring to a plurality of photographed images of the tablet when it is difficult to determine the propriety of the tablet by using only one photographed image of the tablet, for example. In the dispensing detail area A322, a correct image of the tablet and the photographed image of the tablet are displayed side by side, so that a pharmacist can easily determine propriety of the tablet by comparing the registered image of the tablet and the photographed image of the tablet with each other.

For example, in the dispensing detail area A322 illustrated in Fig. 12A, as photographed images of the tablets folded in the medicine packet 451 of the first packet, photographed images of the corresponding three tablets of "Transamin capsule 250 mg", "Cepharanthine tablet 1 mg", and "Reflex tablets 15 mg" are displayed one by one. When the tablet is a flat tablet, two images of the respective front and back surfaces of the tablet may be displayed, and when the tablet is in the shape of a capsule tablet or the like, two respective images of a surface including identification information on the tablet and a surface including no identification information of the tablet may be displayed.

As illustrated in Fig. 13, in the dispensing detail area A322, when a result of the automatic inspection process of a medicine included in the prescription data displayed in the inspection detail screen D303 is an error, backgrounds of one or more of regions A314 and A315 corresponding to the medicine are displayed in a predetermined fifth specific color such as red. This enables a user to easily find out a medicine in which a result of the automatic inspection process is an error by checking positions of the areas A314 and A315.

In an inspection result area A313, there are displayed results of the automatic inspection process for all medicines included in the prescription data displayed in the inspection detail screen D303. The result of the automatic inspection process displayed in the inspection result area A313 is an example of the dispensing result information. Specifically, in the example illustrated in Fig. 13, "NG" indicating that a result of the automatic inspection process is an error is displayed together with a background in a predetermined sixth specific color such as red, different from the fifth specific color, in the inspection result area A313. In the example illustrated in Fig. 14A, "CHECK" indicating that a result of the automatic inspection process needs to be checked is displayed together with a background in a predetermined seventh specific color such as yellow, different from the fifth and sixth specific colors, in the inspection result area A313.

Particularly, in the dispensing detail area A322, an individual result display area A323 showing an inspection result of the automatic inspection process for each of the tablets included in the prescription data is individually displayed. Specifically, in the example illustrated in FIG. 13, "○" indicating that a result of an inspecting process is appropriate is displayed for "Transamin capsule 250 mg" and "Cepharantht tablet 1 mg", and "×" indicating that an error occurs in the result of the inspection process is displayed for "Reflex tablets 15 mg". This enables a pharmacist to easily grasp the tablet causing the error as a result of the inspection process, for example. Information indicating propriety of the inspection process is also an example of the dispensing result information.

When a selection operation of photographed images of the tablet displayed in the dispensing detail area A322 is performed, as illustrated in Figs. 18A and 18B, the control unit 21 is capable of causing the client terminal 3 to display an enlarged screen D341 for displaying a photographed image of the tablet, to be subjected to the selection operation, in an enlarged manner as an enlarged image P343. In the enlarged screen D341, while an enlarged image P342 obtained by enlarging a correct image corresponding to the tablet to be subjected to the selection operation and the enlarged image P 343 are displayed, only the enlarged image P343 may be displayed.

As illustrated in Fig. 12A, when an inspection result by the automatic inspection process is appropriate, the inspection detail screen D303 displays "waiting for approval" in the inspection result area A313 as an inspection result of the automatic inspection process. In addition, the inspection detail screen D303 displays an approval key K311 used to perform an operation for approving an inspection result being displayed.

Meanwhile, as illustrated in Fig. 13, when an inspection result by the automatic inspection process is an error, the inspection detail screen D303 displays "NG" indicating that an inspection result of the automatic inspection process is an error, in the inspection result area A313.

As illustrated in Fig. 14A, when an inspection result of by the automatic inspection process needs to be checked, the inspection detail screen D303 displays "CHECK" indicating that an inspection result of the automatic inspection process is an error, in the inspection result area A313.

In particular, when identification information on the tablet cannot be acquired in the automatic inspection process, or when there is no character printing or no engraved mark exists on the tablet like the tablet "Tictack" illustrated in Fig. 14B, the control unit 21 causes a display for indicating that that all of the medicine packets 451 in which the tablet is housed need to be checked. In addition, when the tablet is a half-tablet, a quarter tablet, or the like, having a size less than that of one tablet, when the tablet is a predetermined image inspecting exclusion medicine such as a transparent and a translucent encapsulated tablet, or when the tablet is an unregistered medicine meaning that the correct image of the tablet is not registered in the medicine master, it is also conceivable that the control unit 21 treats an inspection result of the tablet as a result necessary to be checked regardless of a result of the automatic inspection process.

When an inspection result of the automatic inspection process needs to be checked, the approval key K311 is operable as illustrated in Figs. 14A and 14B. However, when an inspection result of the automatic inspection process is an error, the approval key K311 is inoperable due to grayed out or the like as illustrated in Fig. 13.

The inspecting detail screen D303 also displays operation keys K312 to K314. The operation key K312 is an operation key for starting a re-dispensing operation for re-performing the dispensing processes for a timing of dosage corresponding to one or more medicine packets 451. When the reissue key K312 is operated, the control unit 21 causes a reissue operation screen D304 for setting a method for re-performing the dispensing processes to be displayed. As described above, in the inspection support system 1, after the display processing unit 211 causes the inspection detail screen D303 to display the prescription-related information and the dispensing result information, it is possible to cause the dispensing processes to be re-performed for one or more of the timings of dosage in response to a predetermined re-dispensing operation. Meanwhile, in the inspection support system 1, it is also conceivable to consider a configuration capable of re-performing the dispensing processes for one or more of the timings of dosage in response to a predetermined re-dispensing operation after the display processing unit 211 causes the inspection detail screen D303 to display at least the dispensing result information, as an embodiment of the present invention. The prescription-related information and the dispensing result information in the inspection detail screen D303 do not need to be displayed in the same screen all at once, and thus it is also conceivable that the information is sequentially displayed or displayed by a scroll operation. Fig. 15 illustrates an example of the reissue operation screen D304.

As illustrated in Fig. 15, the reissue operation screen D304 enables "All packets", "Only CHECK packet", "Only NG packet", "CHECK and NG packets", and "Designated packet" to be selected as a target for re-performing the dispensing processes. When the "All packets" is selected, all the medicine packets 451 are targeted, and when the "Only CHECK packet" is selected, only the medicine packet 451 having an inspection result necessary to be checked is targeted. In addition, when the "Only NG packet" is selected, the medicine packet 451 having an inspection result of an error is targeted, and when the "CHECK and NG packets" is selected, the medicine packet 451 having an inspection result of an error and the medicine packet 451 having an inspection result necessary to be checked are targeted. Further, the "Designated packet" enables the medicine packet 451 to be re-performed to be arbitrarily designated, and enables continuous designation or individual designation, for example. When the medicine packet 451 to be re-formed has been already designated in another screen, identification information on the designated medicine packet 451, such as its medicine packet number, is displayed in an input section of the designated packet.

The reissue operation screen D304 displays a reissue key K315 for starting to perform reissue, and the control unit 21 transmits a control command including the re-performing data, the re-performing command, and the like, for re-performing the dispensing processes of the designated medicine packet 451, to the tablet dispensing device 4 in response to an operation of the reissue key K315. The processing described above is performed by a re-performing processing unit 213 of the control unit 21. This allows the tablet dispensing device 4 to re-perform the dispensing processes for a timing of dosage corresponding to the medicine packet 451 designated in the reissue operation screen D304, according to the control command.

Returning to the description of Fig. 13, the operation key K313 is an operation key for displaying the manual dispensing checking screen D5 that displays a manual dispensing image corresponding to the prescription data currently being displayed. The operation key K313 is operable when the manual dispensing unit 42 is used in the dispensing processes, and is inoperable due to grayed out or the like when the manual dispensing unit 42 is not used in the dispensing processes. When the operation key K313 is operated, the control unit 21 causes a display of the manual dispensing checking screen D5 (refer to Fig. 17) in which the manual dispensing image is to be displayed. As a result, when an inspection result of the dispensing processes by the tablet dispensing device 4 is an error, a user can easily check whether the error is caused by a wrong dispensing of a tablet to the manual dispensing unit 42 while
checking the manual dispensing image taken by the camera 464 for the dispensing of a tablet in the tablet dispensing device 4.

Fig. 17 illustrates an example of the manual dispensing checking screen D5. The manual dispensing checking screen D5 illustrated in Fig. 17 displays a manual dispensing unit image area A51 and an operation instruction area A52, together with the basic information area A31 and the prescription information area A35. The manual dispensing unit image area A51 displays a manual dispensing image obtained by photographing the manual dispensing unit 42 from above by the camera 464. This enables a user to check a state immediately after manual dispensing of a tablet to the manual dispensing unit 42. The operation instruction area A52 displays an instruction of operation contents in the manual dispensing unit image area A51. For example, the operation instruction area A52 displays the fact that an image is enlarged by the pinch-out operation, and is reduced in size by the pinch-in operation, together with a finger gesture and an image.

Returning to the description of Fig. 13, the operation key K314 is an operation unit for changing a display size of a list display of an inspection result in the inspection detail screen D303. When the display size is changed by an operation of the operation key K314, the control unit 21 changes the number of the medicine packets 451 to be simultaneously displayed in the inspection detail screen D303. In the operation key K314, four types of display sizes of "large", "medium", "small", and "minimum" can be arbitrarily selected. As a result, when referring to a result of the automatic inspection process, a user can check each of the photographed images in an enlarged manner, or can select a display mode suitable for a purpose such as grasping the overall result of the automatic inspection process.

As described above, when a photographed image of a specific tablet in a specific one of the medicine packets 451 is selected in the inspection detail screen D303, the control unit 21 causes the enlarged screen D341 corresponding to the tablet to be displayed. The enlarged screen D341 is illustrated in each of Figs. 18A and 18B. As illustrated in Figs. 18A and 18B, the enlarged screen D341 displays an enlarged image P342 corresponding to a correct image, and an enlarged image P343 of the photographed image corresponding to the selected tablet, side by side. In addition, the control unit 21 may cause the enlarged screen D34 to be displayed only for a photographed image of a tablet whose inspection result of the automatic inspection process is an error or needs to be checked, among the photographed images, or may enable the enlarged screen D34 to be displayed for all photographed images of tablets.

Further, the control unit 21 enables the enlarged screen D341 to display the enlarged image P343 while sequentially switching the plurality of photographed images corresponding to the tablet at predetermined intervals, as illustrated in Figs. 18A and 18B, for example. More specifically, when the enlarged screen D341 is displayed, the control unit 21 starts switching the enlarged image P343. This enables a user to determine propriety of the tablet with reference to the plurality of photographed images obtained by photographing an outer periphery of the tablet from different angles.

The enlarged screen D341 displays operation keys K361 to K364. When the operation key K361 is operated, the control unit 21 temporarily stops automatic switching of the enlarged image P343, and restarts the automatic switching of the enlarged image P343 when the operation key K361 is operated again. It is conceivable that the control unit 21 may start the automatic switching of the enlarged image P343 by operating the operation key K361 after the enlarged screen D341 is displayed.

When the operation key K362 is operated in the case where an inspection result of the automatic inspection process of the tablet being displayed needs to be checked or is an error, the control unit 21 causes the fact that the tablet is determined to be appropriate by visual check to be stored as an inspection result of the tablet. In addition, when the operation key K363 is operated, the control unit 21 causes the medicine packet 451 housing the tablet being displayed to be selected and stored as a target of the reissue. It is conceivable that the tablet or the medicine packet 451 selected as a target of the reissue is displayed in the inspection detail screen D303 while being associated with additional information A324 such as a character or a symbol indicating a target to be reissued, as illustrated in Fig. 13.

Further, when the operation key K364 is operated, the control unit 21 causes the fact of performing treatment such as manual replacement of tablets housed in the medicine packet 451, including the tablet being displayed, to be stored. It is conceivable that even in this case, the tablet selected as a target of the manual treatment is displayed in the inspection detail screen D303 while being associated with additional information A325 such as a character or a symbol indicating a target of the manual treatment, as illustrated in Fig. 14A. Only any one of the operation keys K362 to K364 is to be in a selected state, and when any one of them is selected, remaining two keys each are to be in a non-selected state.

In the user master, it is conceivable that processing authority in the enlarged screen D341 is set as the processing authority corresponding to a user or a user group. Each of the user groups is used to classify one or more of users into a general pharmacist, a management pharmacist, a technician, and the like, for example, and the processing authority is set for each of the user groups. Specifically, in the user master, it is conceivable that operability of each of the operation keys K362 to K364 is set for each user or for each user group. Then, the control unit 21 switches the validity and invalidity of each of the operation keys K362 to K364 according to processing authority corresponding to a currently logged-in user. This enables treatable contents for the inspection result to be managed for each user or for each user group.

As illustrated in Figs. 13 and 14A, when inspection results of the automatic inspection process includes an inspection result of an error or that necessary to be checked, the control unit 21 causes the inspection detail screen D303 to display together a tablet having an inspection result of the automatic inspection process, being "OK", and a tablet having an inspection result of the automatic inspection process, being an error or necessary to be checked. When an error tablet is included as an inspection result of the automatic inspection process, "NG" is displayed in the inspection result area A313 as illustrated in Fig. 13. When an error tablet is not included and a tablet necessary to be checked is included, as an inspection result of the automatic inspection process, "CHECK" is displayed in the inspection result area A313, as illustrated in FIG. 14A. In addition, the individual result display area A323 corresponding to a tablet having the inspection result being an error displays a mark "×" indicating being an error, as illustrated in FIG. 13, and the individual result display area A323 corresponding to a tablet having the inspection result being appropriate displays a mark "○" indicating being appropriate, as illustrated in Fig. 12A. As illustrated in Fig. 14A, the individual result display area A323 corresponding to a tablet having an inspection result being necessary to be checked displays a mark "Δ" indicating being necessary to be checked.

Meanwhile, when the number of tablets corresponding to each timing of dosage is counted by the passage detection sensor 474 in the tablet dispensing device 4, it is also conceivable that the control unit 21 causes the inspection detail screen D303 to display the number of tablets. This enables a user to easily grasp that there is no problem in the number of tablets. When the tablet dispensing device 4 includes a photographing unit for photographing the medicine packet 451 after the tablet is dispensed in the dispensing unit 45, it is also conceivable that the control unit 21 causes the detail screen D303 to display a photographed image of the tablet after being dispensed.

### <Step S39>

After that, in step S39, the control unit 21 performs the re-performing control process for causing the tablet dispensing device 4 to re-perform a part or all of the dispensing processes. Fig. 19 illustrates an example of the re-performing control process.

### [Re-performing Control Process]

The re-performing control process performed by the control unit 21 will be described with reference to Fig. 19.

### <Steps S51 to S60>

In steps S51 to S60, processing for re-performing a part or all of the dispensing processes performed by the tablet dispensing device 4 for the prescription data is performed. Here, there is described a case where the dispensing process is re-performed by the tablet dispensing device 4 that has performed the dispensing processes. Meanwhile, it is also conceivable that the control unit 21 causes a tablet dispensing device 4 different from the tablet dispensing device 4, which has performed the dispensing processes for the prescription data, to re-perform a dispensing process for one or more of timings of dosage in response to a user's operation or automatically. This causes the different tablet dispensing devices 4 to re-perform the dispensing processes, so that occurrence of an error or requiring checking in the dispensing processes may be avoided in some cases. The control unit 40 may re-perform a part or all of the dispensing processes in response to a user's operation in the tablet dispensing device 4. In this case, the inspection detail screen D303 and the reissue operation screen D304 are also displayed in the operation display unit 48 of the tablet dispensing device 4, and the control unit 40 re-performs a dispensing process for one or more of timings of dosage in response to a user's re-dispensing operation on the operation display unit 48.

### <Step S51>

In step S51, the control unit 21 determines whether an all-reissue operation is performed in the reissue operation screen D304. More specifically, when the "All packets" is selected in the reissue operation screen D304 and the operation key K315 is operated, the control unit 21 determines that the all-reissue operation is performed. When determining that the all-reissue operation is performed (Yes at S51), the control unit 21 shifts processing to step S52, and shifts the processing to step S53 when the all-reissue operation is not performed (No at S51).

### <Step S52>

In step S52, the control unit 21 performs reissue processing for transmitting a control command, for performing all of the dispensing processes corresponding to the prescription data to be currently inspected, to the tablet dispensing device 4 that has performed the dispensing process. For example, as the control command, the prescription data is re-input to the tablet dispensing device 4. As a result, in the tablet dispensing device 4, the control unit 40 re-performs the dispensing processes for all of the timings of dosage corresponding to the prescription data, as the second dispensing process..

Then, as described above, the control unit 40 of the tablet dispensing device 4 performs the second photographing step and the second storing step. That is, in the second dispensing process that is a dispensing process re-performed for the prescription data, the control unit 40 causes the camera 462 or 463 to take a second photographed image of each of the tablets to be dispensed and causes the second photographed image to be stored while associating it with the prescription data. In particular, as described above, the control unit 40 causes the second photographed image corresponding to each of the timings of dosage in the dispensing processes to be stored while associating it with each of the timings of dosage. In addition, the control unit 40 performs an automatic inspection process of determining whether the dispensing process corresponding to each of the timings of dosage is appropriate on the basis of the second photographed image and the prescription data, and causes an inspection result of the automatic inspection process to be stored while associating it with the prescription data. In particular, the control unit 40 causes an inspection result of the automatic inspection process for each of the timings of dosage to be stored while associating it with each of the timings of dosage. The control unit 40 also determines whether all of the dispensing processes based on the prescription data is correctly performed in the first dispensing process and one or more of the second dispensing processes, and causes a result of the determination to be stored as an inspection result of the automatic inspection process for the whole of the prescription data while associating it the prescription data.

### <Step S53>

In step S53, the control unit 21 determines whether a reissue operation for only a packet necessary to be checked is performed in the reissue operation screen D304. More specifically, when the control key K315 is operated after "Only CHECK packet" is selected in the reissue operation screen D304, the control unit 21 determines that the reissue operation for only a packet necessary to be checked is performed. When determining that the reissue operation for only a packet necessary to be checked is performed (Yes at S53), the control unit 21 shifts processing to step S54, and shifts the processing to step S55 when the reissue operation for only a packet necessary to be checked is not performed (No at S53).

### <Step S54>

In step S54, the control unit 21 performs reissue processing for transmitting a control command to the tablet dispensing device 4, the control command being used for re-performing only a dispensing process corresponding to a timing of dosage in which a table necessary to be checked occurs, among the dispensing processes corresponding to the prescription data to be currently inspected. As a result, in the tablet dispensing device 4, the control unit 40 re-performs the dispensing process only for some of the timings of dosage in each of which the table necessary to be checked occurs, as the second dispensing process.

Then, the control unit 40 of the tablet dispensing device 4 performs the second photographing step and the second storing step. That is, in the second dispensing process being a dispensing process to be re-performed for the prescription data for some of the timings of dosage, the control unit 40 causes the camera 462 or 463 to take a second photographed image for each tablet to be dispensed, and causes the second photographed image to be stored while associating it with the prescription data. In particular, as described above, the control unit 40 causes the second photographed image corresponding to each of the timings of dosage in the dispensing processes to be stored while associating it with each of the timings of dosage. In addition, the control unit 40 performs an automatic inspection process of determining whether the dispensing process corresponding to each of the timings of dosage is appropriate on the basis of the second photographed image and the prescription data, and causes an inspection result of the automatic inspection process to be stored while associating it with the prescription data. In particular, the control unit 40 causes an inspection result of the automatic inspection process for each of the timings of dosage to be stored while associating it with each of the timings of dosage. The control unit 40 also determines whether all of the dispensing processes based on the prescription data is correctly performed in the first dispensing process and one or more of the second dispensing processes, and causes a result of the determination to be stored as an inspection result of the automatic inspection process for the whole of the prescription data while associating it the prescription data. Hereinafter, even when the reissuing processing in steps S56, S58, and S60 is performed, similar processing is performed by the control unit 40 in the tablet dispensing device 4.

### <Step S55>

In step S55, the control unit 21 determines whether a reissue operation for only an NG packet is performed in the reissue operation screen D304. More specifically, when the control key K315 is operated after "Only NG packet" is selected in the reissue operation screen D304, the control unit 21 determines that the reissue operation for only an NG packet is performed. When determining that the reissue operation for only an NG packet is performed (Yes at S55), the control unit 21 shifts processing to step S56, and shifts the processing to step S57 when the reissue operation for only an NG packet is not performed (No at S55).

### <Step S56>

In step S56, the control unit 21 performs reissue processing for transmitting a control command to the tablet dispensing device 4, the control command being used for re-performing only a dispensing process of a timing of dosage in which an error occurs, among prescriptions corresponding to the prescription data to be currently inspected. As a result, in the tablet dispensing device 4, the control unit 40 re-performs the dispensing process only for some of the timings of dosage in each of which the error occurs, as the second dispensing process.

### <Step S57>

In step S57, the control unit 21 determines whether the reissue operation for an NG packet and a packet necessary to be checked is performed in the reissue operation screen D304. More specifically, when the control key K315 is operated after "CHECK and NG packets" is selected in the reissue operation screen D304, the control unit 21 determines that a reissue operation for only an NG packet and a packet necessary to be checked is performed. When determining that the reissue operation for only an NG packet and a packet necessary to be checked is performed (Yes at S57), the control unit 21 shifts processing to step S58, and shifts the processing to step S59 when the reissue operation for only an NG packet and a packet necessary to be checked is not performed (No at S57).

### <Step S58>

In step S58, the control unit 21 performs reissue processing for transmitting a control command to the tablet dispensing device 4, the control command being used for re-performing only a dispensing process of a timing of dosage in which an error or a tablet necessary to be checked occurs, among prescriptions corresponding to the prescription data to be currently inspected. As a result, in the tablet dispensing device 4, the control unit 40 re-performs the dispensing process only for some of the timings of dosage in each of which the error or the table necessary to be checked occurs, as the second dispensing process.

When various reissuing operations are performed for prescription data for which the dispensing processes has been already re-performed by the tablet dispensing device 4, it is conceivable that the control unit 21 notifies the fact that the dispensing process for the prescription data has been already re-performed to a user by a display in the display unit 24 or the like. It is also conceivable that the control unit 21 does not transmit a control command for re-performing the dispensing process. Meanwhile, after notifying the fact that the dispensing process has been re-performed to a user by a display in the display 24 or the like, the control unit 21 may transmit a control command, for re-performing the dispensing processes corresponding to the reissue operation, to the tablet dispensing device 4 when the user performs a re-performing continuing operation.

### <Step S59>

In step S59, the control unit 21 determines whether a reissue operation for only a designated packet is performed in the reissue operation screen D304. More specifically, when the control key K315 is operated after "Designated packet" is selected in the reissue operation screen D304, the control unit 21 determines that the reissue operation for only a designated packet is performed. When determining that the reissue operation for only a designated packet is performed (Yes at S59), the control unit 21 shifts processing to step S40, and shifts the processing to step S60 when the reissue operation for only a designated packet is not performed (No at S59).

### <Step S60>

In step S60, the control unit 21 performs reissue processing for transmitting a control command to the tablet dispensing device 4, the control command being used for re-performing only a dispensing process corresponding to a timing of dosage designated in the reissue operation screen D304, among the dispensing processes corresponding to the prescription data to be currently inspected. As a result, in the tablet dispensing device 4, the control unit 40 re-performs the dispensing process only for the timing of dosage designated in the reissue operation screen D304, as the second dispensing process.

### <Step S18>

Returning to description of Fig. 7, in step S18, the control unit 21 determines whether an approval operation of a result of the automatic inspection process for the prescription data is performed. Specifically, the control unit 21 determines whether the approval key K311 is operated in the inspection detail screen D303. When determining that the approval operation is performed (Yes at S18), the control unit 21 shifts processing to step S19, and shifts the processing to step S20 when the approval operation is not performed (No at S19).

### <Step S19>

In step S19, the control unit 21 performs an approval process for approving a result of the automatic inspection process for the prescription data. For example, in the approval processing, the control unit 21 caused the storage unit 22 to store the fact that a result of the automatic inspection process for the prescription data is approved and identification information (a name or an ID) of a user being a pharmacist who has performed the approval operation, together with the result of the automatic inspection process, while associating them with the prescription data.

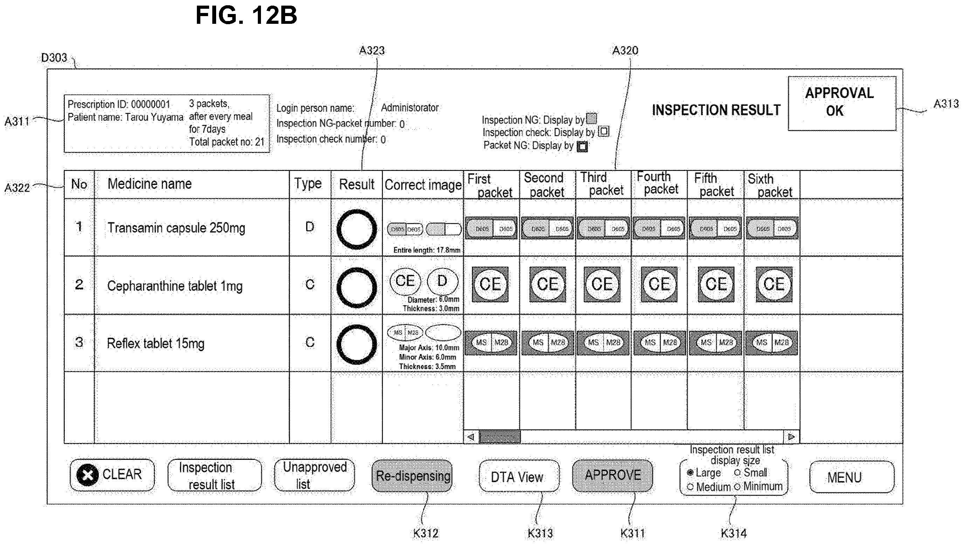

Specifically, when the approval key K311 is operated in the inspection detail screen D303, the control unit 21 receives approval for an inspection result of the automatic inspection process and causes the inspection result to be stored while associating it with the prescription data. As illustrated in Fig. 12B, the control unit 21 also causes "Approval OK" to be displayed in the inspection result area A313 in the inspection detail screen D303, as an inspection result of the automatic inspection process.

### <Step S20>

In step S20, the control unit 21 determines whether a preset inspection finishing operation for finishing the inspection support process is performed. For example, when an operation key corresponding to "End" in the inspection waiting list screen D1 is operated, the control unit 21 determines that the inspection finishing operation is performed. When determining that the inspection finishing operation is performed (Yes at S20), the control unit 21 finishes the inspection support process, and returns processing to the step S12 when the inspection finishing operation is not performed (No at S20).

In the inspection support system 1 configured as described above, the plurality of dispensing processes may be performed for the same prescription data by re-performing the dispensing process. In contrast, in the inspection support system 1 according to the present embodiment, a pharmacist can easily find out propriety of a final dispensing result in consideration of multiple dispensing processes performed by the tablet dispensing device 4, so that it is possible to support an inspection operation by the pharmacist.

Hereinafter, there is described processing of steps S32 to S38 performed when it is determined that the re-performing is performed for the prescription data (Yes at S31) in the inspection display control processing illustrated in FIG. 11. As described above, a dispensing process performed first for the same prescription data may be referred to as a first dispensing process, and the second and subsequent dispensing processes may be referred to as a second dispensing process. In addition, a tablet image taken in the first dispensing process may be referred to as a first photographed image, and a tablet image taken in the second dispensing process may be referred to as a second photographed image.

### <Step S32>

In step S32, the control unit 21 causes the inspection detail screen D303 to be displayed in a combined display state where the first photographed image taken in the first dispensing process and the second photographed image taken in the second splitting process are combined with each other. In particular, the control unit 21 causes the first photographed image and the second photographed image to be displayed in the inspection detail screen D303 in a distinguishable form. That is, the control unit 21 notifies a packet of the medicine packets 451 after dispensing obtained in the first dispensing process, to be replaced with the medicine packet 451 after dispensing obtained in the second dispensing process, to a user according to a difference in the distinguishable form. For example, a background of the first photographed image is displayed in a predetermined first background color, and a background of the second photographed image is displayed in a predetermined second background color. When there is the plurality of second photographed images for the same timing of dosage, each of the second photographed images is displayed in a state distinguishable by a difference in background color or the like.

Specifically, in the inspection detail screen D303 in the combined display state, the control unit 21 causes the second photographed image to be displayed for a timing of dosage having the second photographed image of the timings of dosage included in the prescription data. In addition, in the inspection detail screen D303 in the combined display state, the control unit 21 causes the first photographed image to be displayed for a timing of dosage having no second photographed image of the timings of dosage included in the prescription data. That is, the control unit 21 causes a photographed image taken in a dispensing process performed later to be preferentially displayed. As a result, the control unit 21 causes only any one of the first photographed image and the second photographed image to be displayed for each of the timings of dosage. The configuration in which the first photographed image and the second photographed image are selectively displayed for each of the timings of dosage is merely an example. As a method for displaying the first photographed image taken in the first dispensing process and the second photographed image taken in the second dispensing process by combining the images with each other, both the first photographed image and the second photographed image may be simultaneously displayed for each of the timings of dosage, for example.

Fig. 20 illustrates an example of the inspection detail screen D303 in a first display state where only the first photographed image is displayed as a dispensing result of the first dispensing process. Fig. 21 illustrates an example of the inspection detail screen D303 in the combined display state displayed in step S32. Fig. 22 illustrates an example of the inspection detail screen D303 in a second display state where only the second photographed image is displayed as a dispensing result of the second dispensing process.

As illustrated in Fig. 20, in the first dispensing process, there is an error in an inspection result of each of timings of dosage corresponding to the third packet and the six packet of the first to sixth packets, and there is described an example of a case where the second dispensing process is correctly performed, while designating the timings of dosage corresponding to the third packet and the sixth packet. That is, it is assumed that there is no error in the second dispensing process performed for the third packet and the sixth packet.

In this case, as illustrated in Fig. 21, in the inspection detail screen D303 in the combined display state, the second photographed image is displayed for each of timings of dosage of the third and sixth packets, for which there are the second photographed images, of the timings of dosage included in the prescription data, and the first photographed image is displayed for each of the timings of dosage of the first, second, fourth, and fifth packets, for which there is no second photographed image. The result display area A313 displays characters, "waiting for approval", indicating that the first to sixth packets are correctly performed in the first dispensing process and the second dispensing process.

As a result, a user can easily find out that the first to sixth packets are correctly performed in the first dispensing process and the second dispensing process, or that a final dispensing result is appropriate, while referring to the inspection detail screen D303 in the composite display state. In addition, the inspection detail screen D303 displays the first photographed image and the second photographed image taken when the first dispensing process or the second dispensing process corresponding to each of the timings of dosage is correctly performed, so that it is possible to easily grasp a state of each of the medicine packets 451, to be a final dispensing result of each of the dispensing processes based on the prescription data.

As illustrated in Fig. 21, when the inspection detail screen D303 is displayed in the combined display state, it is conceivable that the control unit 21 performs processing for determining propriety of the medicine packet 451 obtained as a dispensing result in each of the first dispensing process and the second dispensing process, on the basis of code information read out by the code reading unit 27. Specifically, the control unit 21 causes the operation key K311 to be displayed in a grayed out state at the time of starting a display of the inspection detail screen D303. When the code information read out from the medicine packet 451 using the code reading unit 27 of the server 2, the code reading unit 37 of the client terminal 3, or the like, is appropriate, the control unit 21 changes a dispensing result of the record to "modified". As a result, when the inspection history screen D2 is displayed, "NG → modified" is displayed for the record.

For example, when the first photographed image and the second photographed image are combined and displayed in the inspection detail screen D303 for the prescription data, or when the first dispensing process and the second dispensing process are performed for the prescription data, it is conceivable that the control unit 21 causes a check screen D32 to be displayed as illustrated in Fig. 16, the check screen D32 being used for prompting a user to read out code information (one-dimensional code or two-dimensional code) described in the medicine packet 451 obtained in the second dispensing process or the like. After that, when code information corresponding to the prescription data is read out from an empty medicine packet 451 attached to the medicine packet 451 obtained in each of the second dispensing processes based on the prescription data to be currently inspected, the control unit 21 causes the result display area A313 to display characters, "waiting for approval", indicating that an inspection result is appropriate. This ensures that the medicine packets 451 obtained in the second dispensing process are ready. It is also conceivable that the code information is read out from an empty medicine packet 451 attached to the medicine packet 451 obtained in the first dispensing process, and is checked. In addition, when the code information is read out from the medicine packet 451 obtained in the second dispensing process, the control unit 21 may notify a user of a message or the like indicating a position of a packet of the medicine packets 451 obtained in the first dispensing process, the packet being to be replaced with the medicine packet 451 obtained by the second dispensing process.

The inspection detail screen D303 displays the first photographed image and the second photographed image in a distinguishable form, so that a user can easily identify one of the medicine packets 451 obtained in the first dispensing process, the one medicine packet 451 being to be replaced with the medicine packet 451 obtained in the second dispensing process. It is also conceivable that the control unit 21 notifies a user of the medicine packet 451 of the medicine packets 451 after dispensing obtained in the first dispensing process, to be replaced with the medicine packet 451 after dispensing obtained in the second dispensing process, by displaying a message notifying it.

When the second dispensing process is for re-performing all of the first dispensing processes, it is also conceivable that the control unit 21 causes the first photographed image or the second photographed image, when a result of the automatic inspection process is determined to be appropriate, to be preferentially displayed for each of the timings of dosage, between the first photographed image and the second photographed image. This causes a combined display of an image showing that an inspection result of the automatic inspection process corresponding to the first dispensing process is appropriate and an image showing that an inspection result of the automatic inspection process corresponding to the second dispensing process is appropriate, for example.

### <Step S33>

Next, in step S33, the control unit 21 determines whether a display request operation for displaying the inspection detail screen D303 in the first display state is performed. Specifically, the inspection detail screen D303 displays an operation key K41 to be used for an operation for displaying the inspection detail screen D303 in the first display state. When the operation key K41 is operated in step S33, the control unit 21 determines that a display request operation for displaying the inspection detail screen D303 in the first display state is performed. When it is determined that a display request operation for displaying the inspection detail screen D303 in the first display state is performed (Yes at S33), processing proceeds to step S34, and when it is determined that the display request operation for displaying the inspection detail screen D303 in the first display state is not performed (No at S33), the processing proceeds to step S35.

### <Step S34>

In step S34, the control unit 21 causes the inspection detail screen D303 to be displayed in the first display state where only the first photographed image taken in the first dispensing process is displayed. Specifically, as illustrated in Fig. 20, the inspection detail screen D303 in the first display state displays the first photographed image among images of the timings of dosage included in the prescription data, and the result display area A313 displays characters, "NG" indicating that an inspection result of the first dispensing process is an error. That is, the control unit 21 causes the result display area A313 to display an inspection result acquired by determining propriety of the entire dispensing processes for the prescription data.

In addition, the control unit 21 causes a display in the fifth specific color for each of the areas A315 corresponding to a timing of dosage having an inspection result of the automatic inspection process being inappropriate among the first dispensing processes. That is, the control unit 21 causes the result display area A313 to display an inspection result acquired by determining propriety of the dispensing processes for the prescription data, for each of the timings of dosage. The display area A320 corresponding to the timing of dosage may be displayed in the fifth specific color indicating that the inspection result is an error.

### <Step S35>

Next, in step S35, the control unit 21 determines whether a display request operation for displaying the inspection detail screen D303 in the combined display state is performed. Specifically, the inspection detail screen D303 displays an operation key K42 to be used for an operation for displaying the inspection detail screen D303 in the combined display state. When the operation key K42 is operated in step S35, the control unit 21 determines that a display request operation for displaying the inspection detail screen D303 in the combined display state is performed. When it is determined that a display request operation for displaying the inspection detail screen D303 in the combined display state is performed (Yes at S35), processing proceeds to step S36, and when it is determined that the display request operation for displaying the inspection detail screen D303 in the combined display state is not performed (No at S35), the processing proceeds to step S37.

### <Step S36>

In step S36, the control unit 21 causes the inspection detail screen D303 to be displayed in the combined display state. When the inspection detail screen D303 is already displayed in the combined display state, that state is maintained.

### <Step S37>

Next, in step S37, the control unit 21 determines whether a display request operation for displaying the inspection detail screen D303 in the second display state is performed. Specifically, the inspection detail screen D303 displays an operation keys K43 and K44 to be used for an operation for displaying the inspection detail screen D303 in the second display state. When the operation key K43 or K44 is operated in step S37, the control unit 21 determines that a display request operation for displaying the inspection detail screen D303 in the second display state is performed. When it is determined that a display request operation for displaying the inspection detail screen D303 in the second display state is performed (Yes at S37), processing proceeds to step S38, and when it is determined that the display request operation for displaying the inspection detail screen D303 in the second display state is not performed (No at S37), the processing proceeds to step S39.

### <Step S38>

In step S38, the control unit 21 causes the inspection detail screen D303 to be displayed in the second display state where only the second photographed image taken in the second dispensing process is displayed. Specifically, as illustrated in Fig. 22, the inspection detail screen D303 in the second display state displays the second photographed image among images of the timings of dosage included in the prescription data, and the result display area A313 displays characters, "waiting for approval" indicating that the first to sixth packets are correctly performed in the first dispensing process and the second dispensing process.

When the second dispensing process is performed multiple times the prescription data to be currently inspected, the second photographed image corresponding to each of the second dispensing processes is stored in the storage unit 22. Then, the control unit 21 causes the second display state corresponding to each of the second dispensing processes to be individually switched and displayed in response to an operation of each of the operation keys K43 and K44. For example, when the second dispensing process is performed twice, the operation key K43 is operated to cause the inspection detail screen D303 to be displayed in the second display state corresponding to the second dispensing process for the first time, and then the operation key K44 is operated to cause the inspection detail screen D303 to be displayed in the second display state corresponding to the second dispensing process of the second time.

As described above, the inspection support system 1 is capable of displaying the first photographed image and the second photographed image by combining the images, so that a pharmacist can easily find out a final dispensing result in consideration of the first dispensing process and the second dispensing process while referring to the display.

### REFERENCE SIGNS LIST

1: inspection support system
2: server
21: control unit
22: storage unit
23: communication I/F
24: display
25: operation unit
26: drive unit
27: code reading unit
3: client terminal
31: control unit
32: storage unit
33: communication I/F
34: display
35: operation unit
36: drive unit
4: tablet dispensing device (example of prescription apparatus)
40: control unit
41: tablet cassette
42: manual dispensing unit
43: individual dispensing unit
44: rotating unit
45: dispensing unit
46: photographing unit
47: passage detector
5: prescription apparatus
6: host system

## Claims

1. An inspection support system comprising:
a first storage processing unit that stores a first photographed image of a tablet photographed in a first dispensing process in which one or more tablets are wrapped by a packaging material for each timing of dosage on the basis of prescription data, and a second photographed image of the tablet photographed in one or more second dispensing processes that are re-performed for one or more timings of the dosage after the first dispensing process is performed, while associating the images with the prescription data; and
a display processing unit capable of displaying the first photographed image and the second photographed image stored by the first storage processing unit.

2. The inspection support system according to claim 1, further comprising:
an inspection processing unit that performs an automatic inspection process of determining propriety of the dispensing process based on the prescription data on the basis of the first photographed image or the second photographed image; and
a second storage processing unit that stores an inspection result of the automatic inspection process based on the first photographed image by the inspection processing unit and an inspection result of the automatic inspection processing based on the second photographed image by the inspection processing unit, while associating the inspection results with the prescription data, wherein
the display processing unit is capable of displaying the first photographed image and the second photographed image, corresponding to the prescription data, and the inspection results by the automatic inspection process.

3. The inspection support system according to claim 1, further comprising:
an inspection processing unit that performs an automatic inspection process of determining propriety of the dispensing process based on the prescription data for each timing of dosage on the basis of the first photographed image or the second photographed image; and
a second storage processing unit that stores an inspection result of the automatic inspection process based on the first photographed image for each timing of dosage by the inspection processing unit and an inspection result of the automatic inspection processing based on the second photographed image for each timing of dosage by the inspection processing unit, while associating the inspection results with each timing of dosage, wherein
the display processing unit is capable of displaying the first photographed image and the second photographed image, corresponding to each timing of dosage in the prescription data, and the inspection result for each timing of dosage.

4. The inspection support system according to any one of claims 1 to 3, wherein
the first storage processing unit stores each timing of dosage in the prescription data when the first photographed image or the second photographed image is photographed, while associating each timing of dosage with the first photographed image or the second photographed image.

5. The inspection support system according to any one of claims 1 to 4, wherein
the display processing unit is capable of displaying the first photographed image photographed in the first dispensing process and the second photographed image photographed in the one or more second dispensing processes, while combining the images with each other.

6. The inspection support system according to claim 5, wherein
the display processing unit displays the second photographed image for the timing of dosage at which the second photographed image exists of timings of dosage included in the prescription data, and displays the first photographed image for the timing of dosage at which the second photographed image does not exist.

7. The inspection support system according to claim 6, wherein
the display processing unit displays the first photographed image or the second photographed image corresponding to each timing of dosage included in the prescription data, while displaying the image side by side for each identical kind of tablets along a predetermined direction.

8. The inspection support system according to claim 7, wherein
the display processing unit displays only one of the first photographed image and the second photographed image for each timing of dosage.

9. The inspection support system according to any one of claims 5 to 8, wherein
the display processing unit is capable of switching among a composite display state in which the first photographed image and the second photographed image are combined to be displayed, a first display state in which only the first photographed image of the first photographed image and the second photographed image is displayed, and a second display state in which only the second photographed image of the first photographed image and the second photographed image is displayed.

10. The inspection support system according to claim 9, wherein
when the plurality of second dispensing processes is performed, the display processing unit is capable of displaying a plurality of the second display states in each of which the second photographed image corresponding to each of the second dispensing processes is displayed, while switching among the second display states.

11. The inspection support system according to any one of claims 1 to 10, further comprising:
a notification processing unit that notifies a packaging material to be replaced with the packaging material after dispensing obtained in the second dispensing process, among the packaging material after dispensing obtained in the first dispensing process.

12. The inspection support system according to any one of claims 1 to 11, wherein
the first storage processing unit associates the first photographed image and the second photographed image with prescription identification information for identifying the prescription data.

13. The inspection support system according to any one of claims 1 to 12, further comprising one or more tablet dispensing devices capable of performing the first dispensing process and the second dispensing process.

14. The inspection support system according to claim 13, wherein
at least one of the tablet dispensing devices includes the display processing unit.

15. A tablet dispensing device comprising:
a dispensing unit that folds one or more tablets with a packaging material on the basis of prescription data;
a photographing unit that is used for photographing the tablets dispensed by the dispensing unit; and
a first storage processing unit that stores a first photographed image of the tablets, photographed by the photographing unit in a first dispensing process in which one or more tablets are packaged with the packaging material for each timing of dosage by the dispensing unit on the basis of the prescription data, and a second photographed image of the tablets, photographed by the photographing unit in one or more second dispensing processes that are re-performed for the one or more timings of dosage after the first dispensing process is performed, while associating the images with the prescription data.

16. The tablet dispensing device according to claim 15, wherein
the first storage processing unit erases one or both of the previous first and second photographed images when storing the second photographed image.

17. A dispensing control program causing a processor mounted in a tablet dispensing device including a dispensing unit that folds one or more tablets with a packaging material on the basis of prescription data, and a photographing unit that is used to photograph the tablets dispensed by the dispensing unit, to perform:
a first photographing step in which, in a first dispensing process in which one or more tablets are packaged with a packaging material for each timing of dosage, the tablets are photographed using the photographing unit;
a first storage step in which a first photographed image photographed in the first photographing step is stored while being associated with the prescription data;
a second photographing step in which the tablets are photographed using the photographing unit in one or more second dispensing processes that are re-performed for the one or more timings of dosage after the first dispensing process is performed; and
a second storage step in which a second photographed image photographed in the second photographing step is stored while being associated with the prescription data.

18. An inspection support system comprising:
a display processing unit that causes a display to display prescription-related information including information on at least a part of the prescription data, and dispensing result information obtained before a dispensing process or after the dispensing process to identify a kind of each of medicines, after the dispensing process in which one or more medicines are packaged with a packaging material for each timing of dosage on the basis of prescription data is performed; and
a re-performing processing unit that causes the dispensing process to be re-performed for the one or more timings of dosage in accordance with a predetermined re-dispensing operation after the prescription-related information and the dispensing result information are displayed by the display processing unit.

19. The inspection support system according to claim 18, wherein
the display processing unit causes at least a dose or usage in the prescription data to be displayed.

20. The inspection support system according to claim 18 or 19, wherein
the display processing unit causes at least a medicine name or a patient name in the prescription data to be displayed.

21. An inspection support system comprising:
a display processing unit that causes a display to display at least dispensing result information obtained before a dispensing process or after the dispensing process to identify a kind of each of medicines, after the dispensing process in which one or more medicines are packaged with a packaging material for each timing of dosage on the basis of prescription data are performed; and
a re-performing processing unit that causes the dispensing process to be re-performed for the one or more timings of dosage in accordance with a predetermined re-dispensing operation after the prescription-related information and the dispensing result information are displayed by the display processing unit.

22. The inspection support system according to any one of claims 18 to 21, further comprising any one of or both of: a photographing unit that photographs the medicine before the dispensing process or after the dispensing process, as a result of the dispensing process; and a component detector that detects components of the medicine before the dispensing process or after the dispensing process, as a result of the dispensing process, wherein
the dispensing result information includes a photographed image of the medicine photographed by the photographing unit or a component detection result detected by the component detector.

23. An inspection support system comprising:
a re-performing processing unit that is capable of re-performing a dispensing process for one or more timings of dosage after the dispensing process in which one or more tablets are packaged with a packaging material for each timing of dosage on the basis of prescription data is performed;
a verification processing unit that verifies the prescription data and a result of the dispensing process; and
a storage processing unit that stores information indicating how many times the dispensing process for each prescription data or for each timing of dosage is performed, for each time of the dispensing process, and a verified result of the dispensing process, while associating the information and the result with each other.

24. The inspection support system according to claim 23, further comprising a display processing unit capable of displaying a verified result of the dispensing process for each timing of dosage in each of the dispensing processes, stored in the storage processing unit.
